# EUROPEAN PATENT SPECIFICATION

(11) **EP 3 538 159 B1**
(45) Date of publication and mention of the grant of the patent: **05.04.2023**
(21) Application number: 17835491.6
(22) Date of filing: 10.11.2017
(51) Int. Cl.: A61K 31/575, A61P 1/16, A61P 3/10

(54) **INHIBITION OF FIBROSIS IN NON-ALCOHOLIC FATTY LIVER DISEASE PATIENTS**
HEMMUNG VON FIBROSE BEI PATIENTEN MIT NICHT ALKOHOLBEDINGTER FETTLEBER
INHIBITION DE LA FIBROSE CHEZ DES PATIENTS ATTEINTS D'UNE STÉATOSE HÉPATIQUE NON ALCOOLIQUE

(30) Priority: 10.11.2016 US 201662420009 P; 22.03.2017 US 201762475132 P
(43) Date of publication of application: 18.09.2019
(73) Proprietor: Galmed Research and Development Ltd., 6578317 Tel Aviv (IL)
(72) Inventor: HAYARDENY-NISSIMOIV, Liat, 69391 Tel Aviv (IL); GORFINE, Tali, 6494210 Tel Aviv (IL); BAHARAFF, Allen, 66215441 Tel Aviv (IL); MATO DE LA PAZ, Jose, M., 48992 Getxo (ES)
(74) Representative: Pearl Cohen Zedek Latzer Baratz UK LLP
(86) International application number: PCT/IB2017/001535
(87) International publication number: WO 2018/087600

(56) References cited:
- WO-A1-2014/093711
- WO-A1-2015/083164
- WO-A1-2016/112305
- WO-A1-2017/017677
- RIFAAT SAFADI ET AL: "The Fatty Acid-Bile Acid Conjugate Aramchol Reduces Liver Fat Content in Patients With Nonalcoholic Fatty Liver Disease", CLINICAL GASTROENTEROLOGY AND HEPATOLOGY, vol. 12, no. 12, 9 May 2014 (2014-05-09), pages 2085-2091.e1, XP055350035, US ISSN: 1542-3565, DOI: 10.1016/j.cgh.2014.04.038
- FRANCESCA MARIA TROVATO ET AL: "4Ps medicine of the fatty liver: the research model of predictive, preventive, personalized and participatory medicine?recommendations for facing obesity, fatty liver and fibrosis epidemics", EPMA JOURNAL, BIOMED CENTRAL LTD, LONDON, UK, vol. 5, no. 1, 7 December 2014 (2014-12-07), page 21, XP021208395, ISSN: 1878-5085, DOI: 10.1186/1878-5085-5-21
- M. IRUARRIZAGA-LEJARRETA ET AL: "Role of Aramchol in Steatohepatitis and Fibrosis in Mice", HEPATOLOGY COMMUNICATIONS, vol. 1, no. 9, 3 November 2017 (2017-11-03), pages 911-927, XP002778683,

## Description

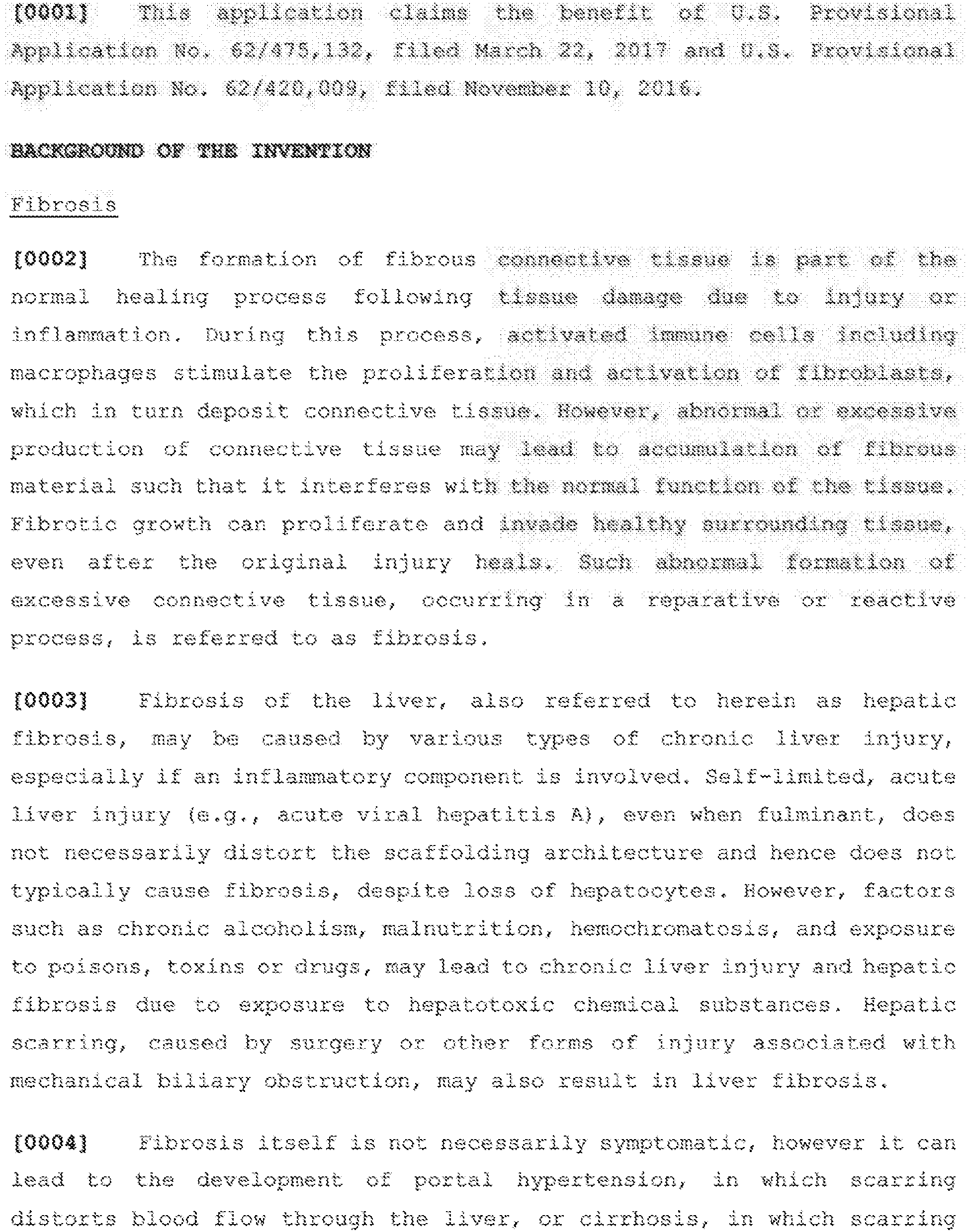

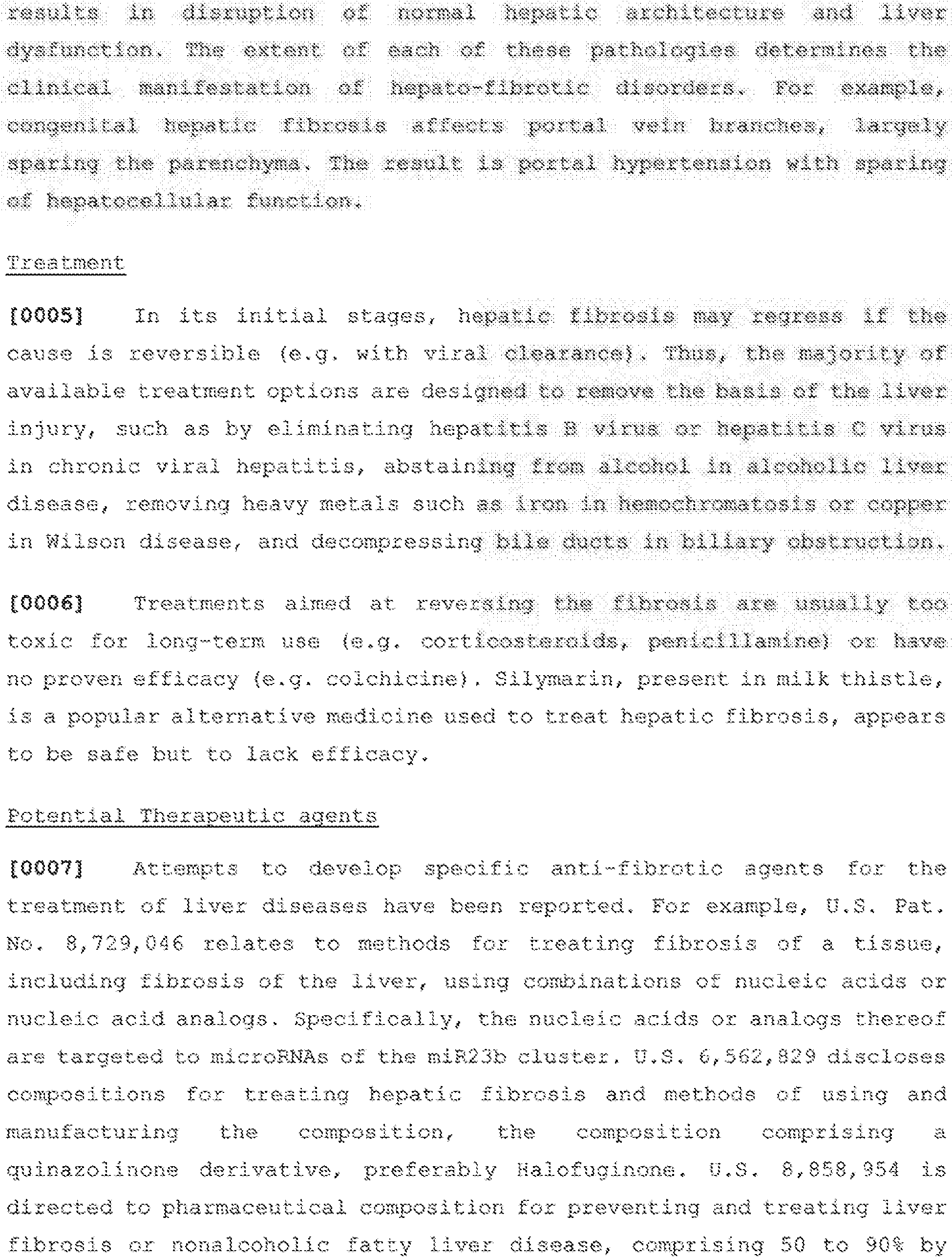

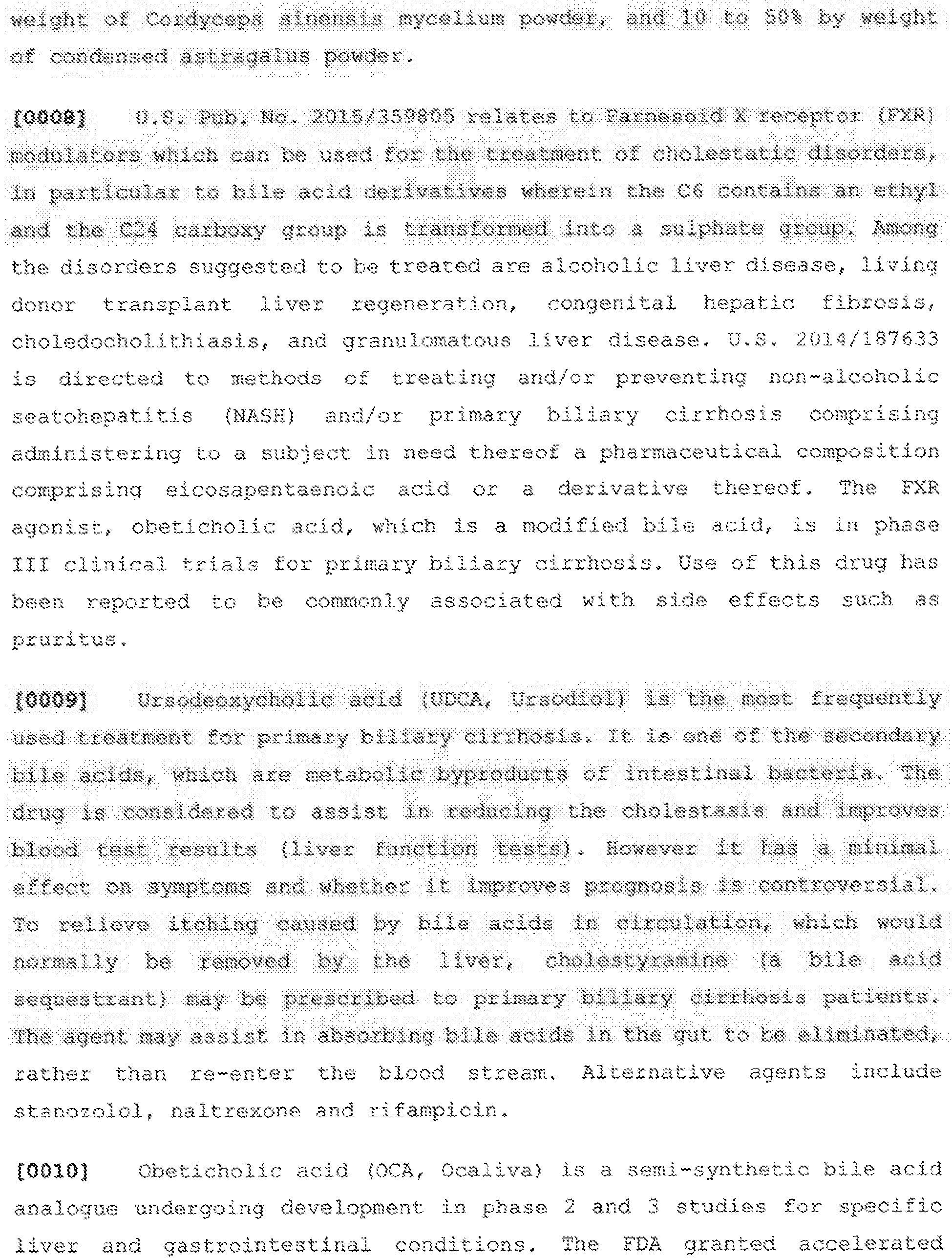

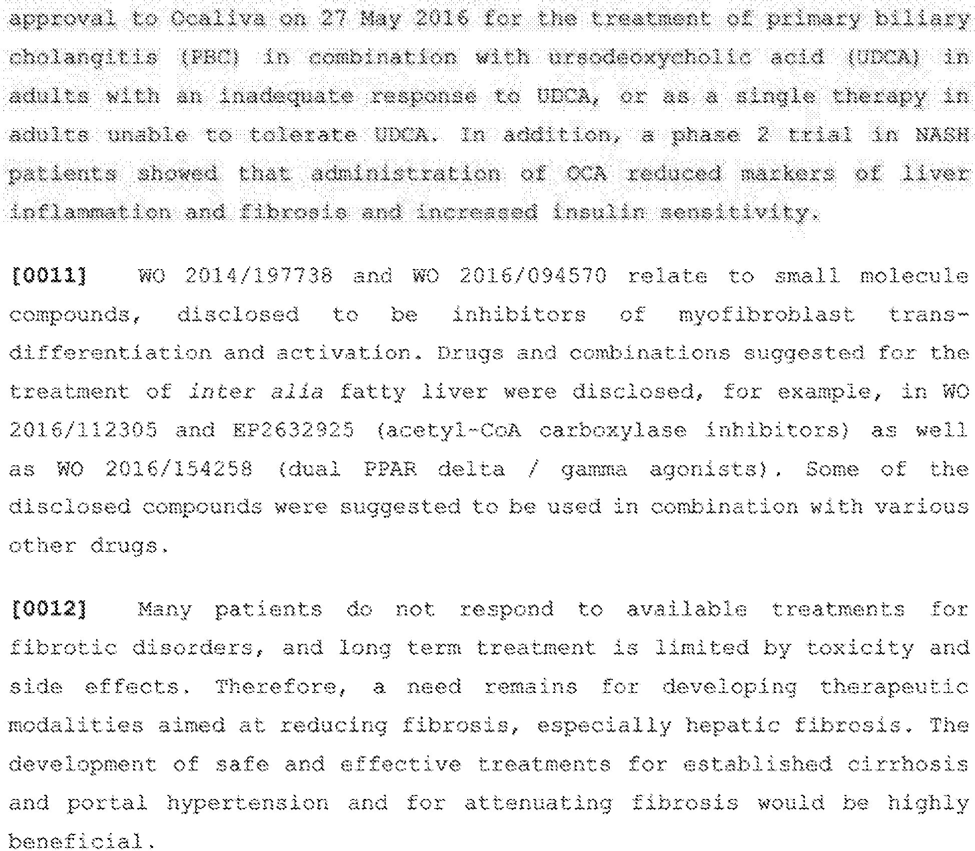

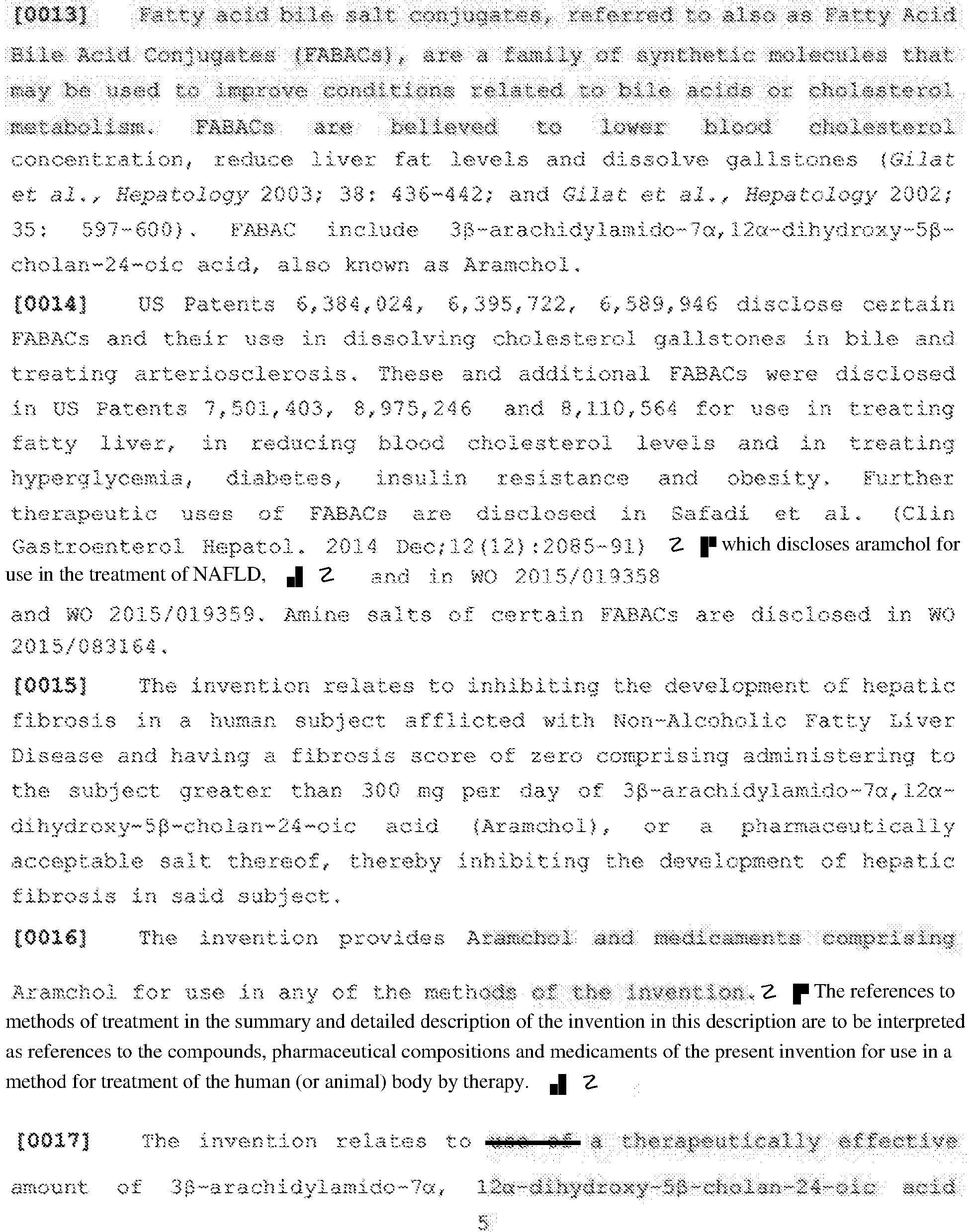

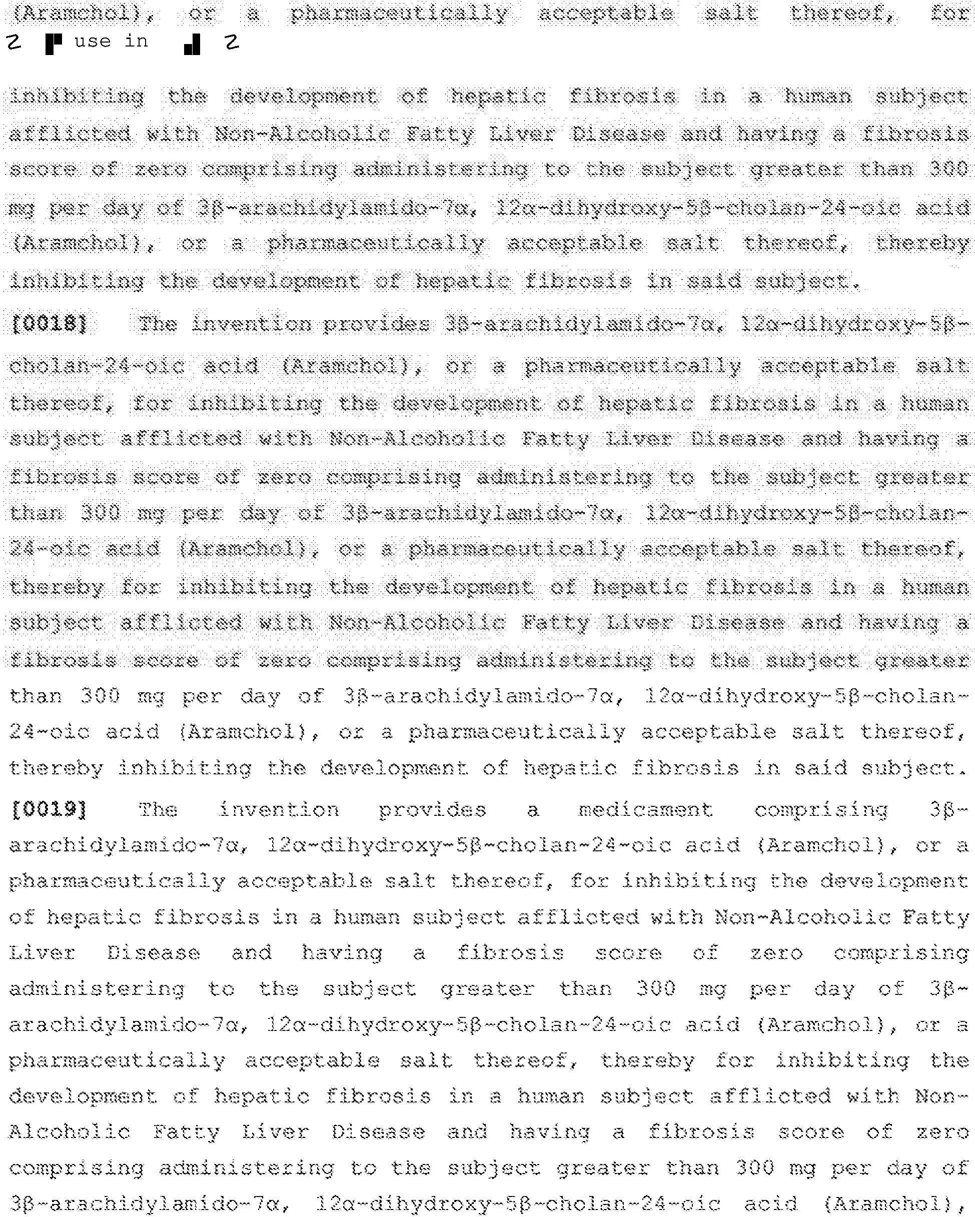

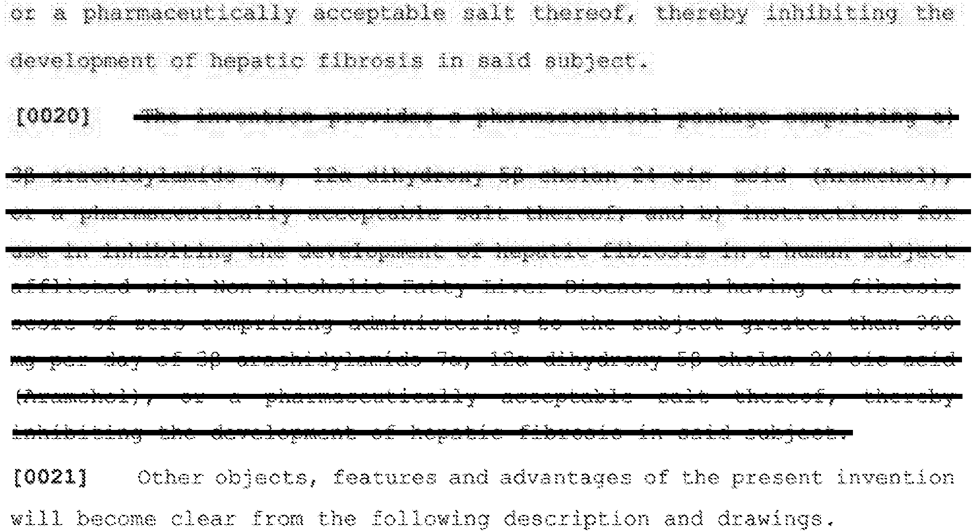

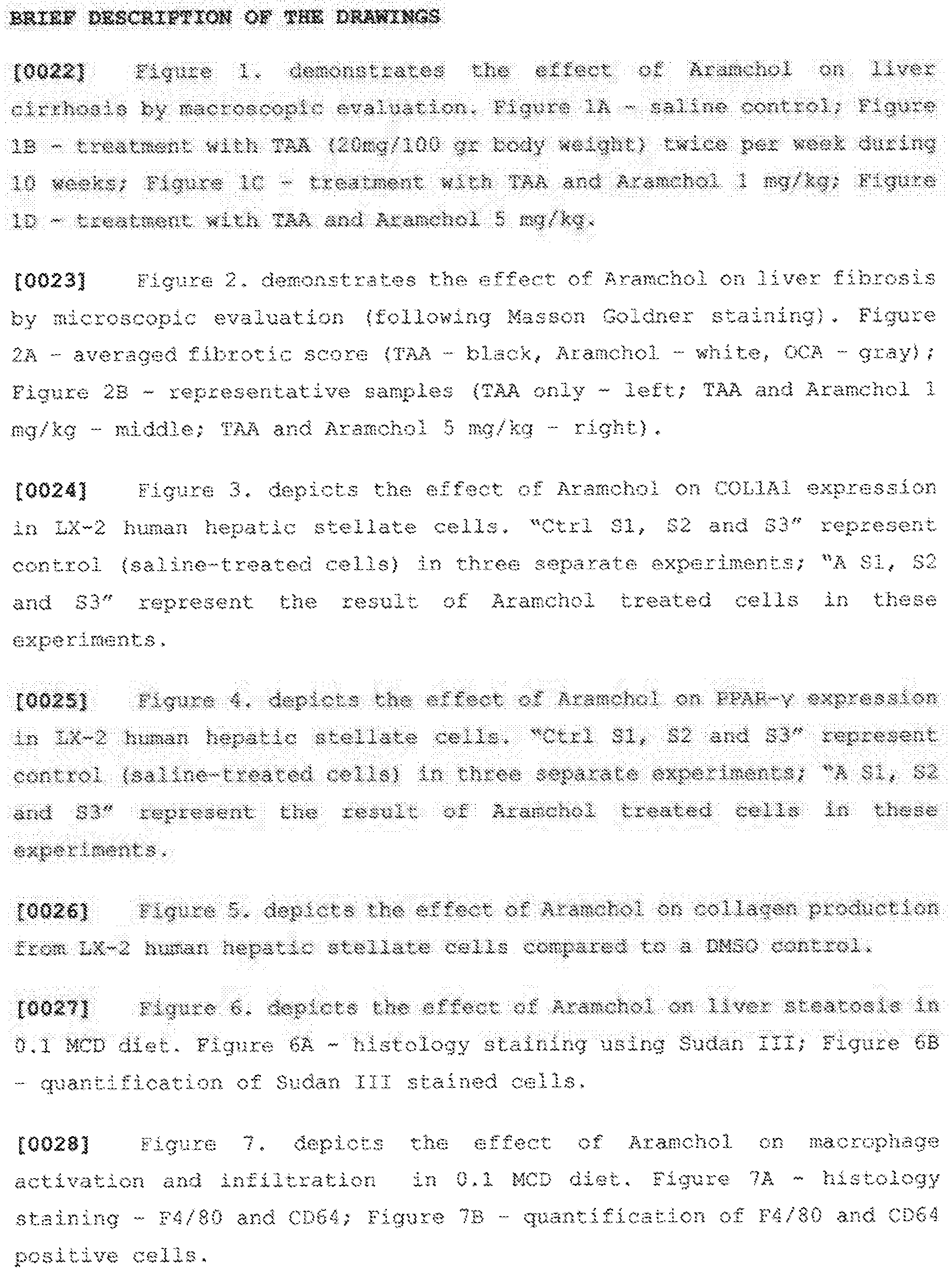

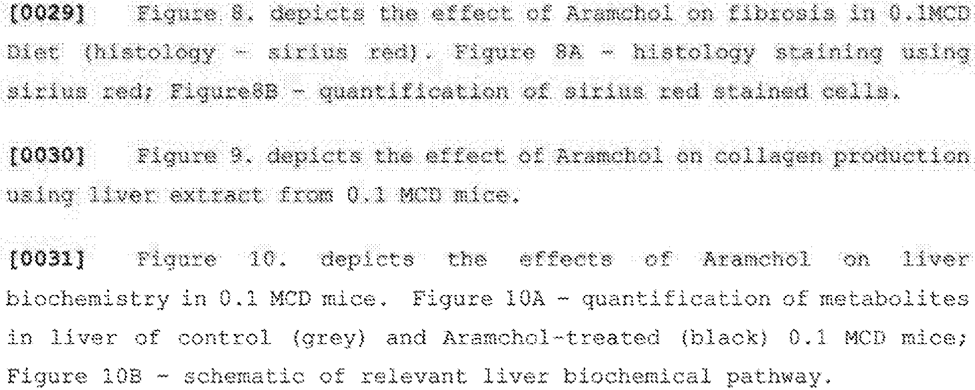

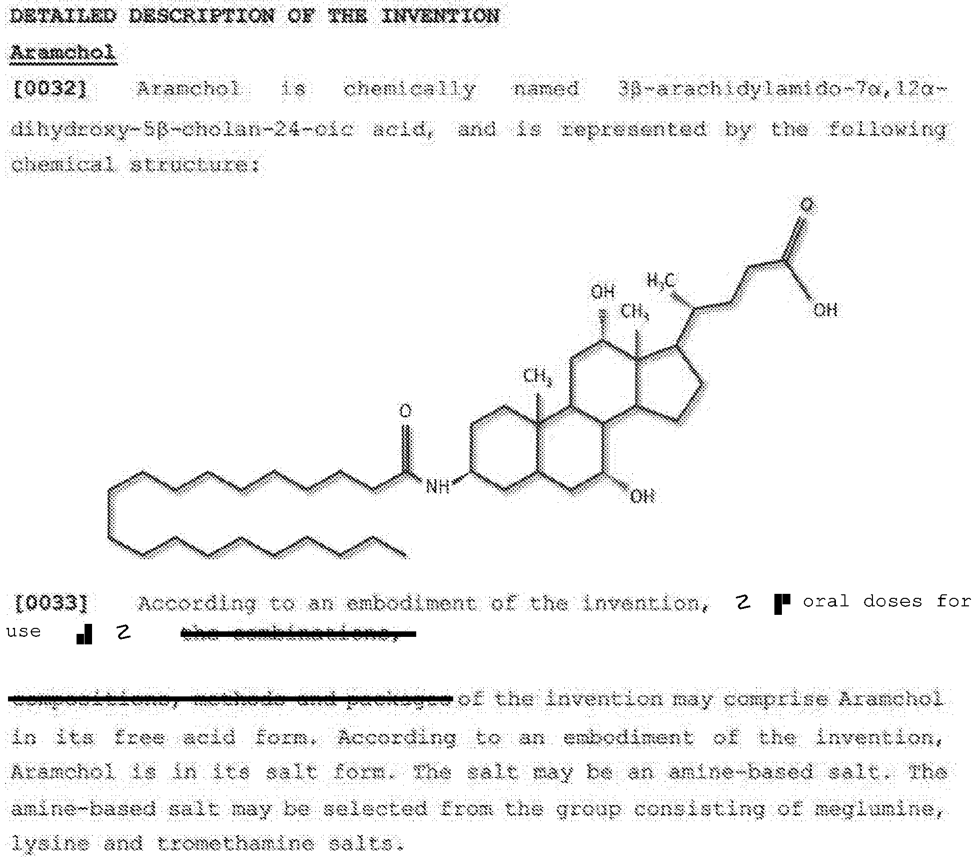

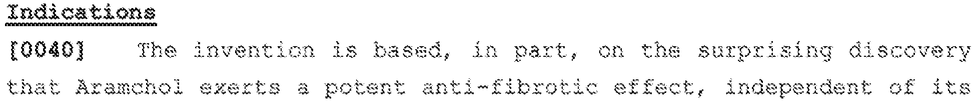

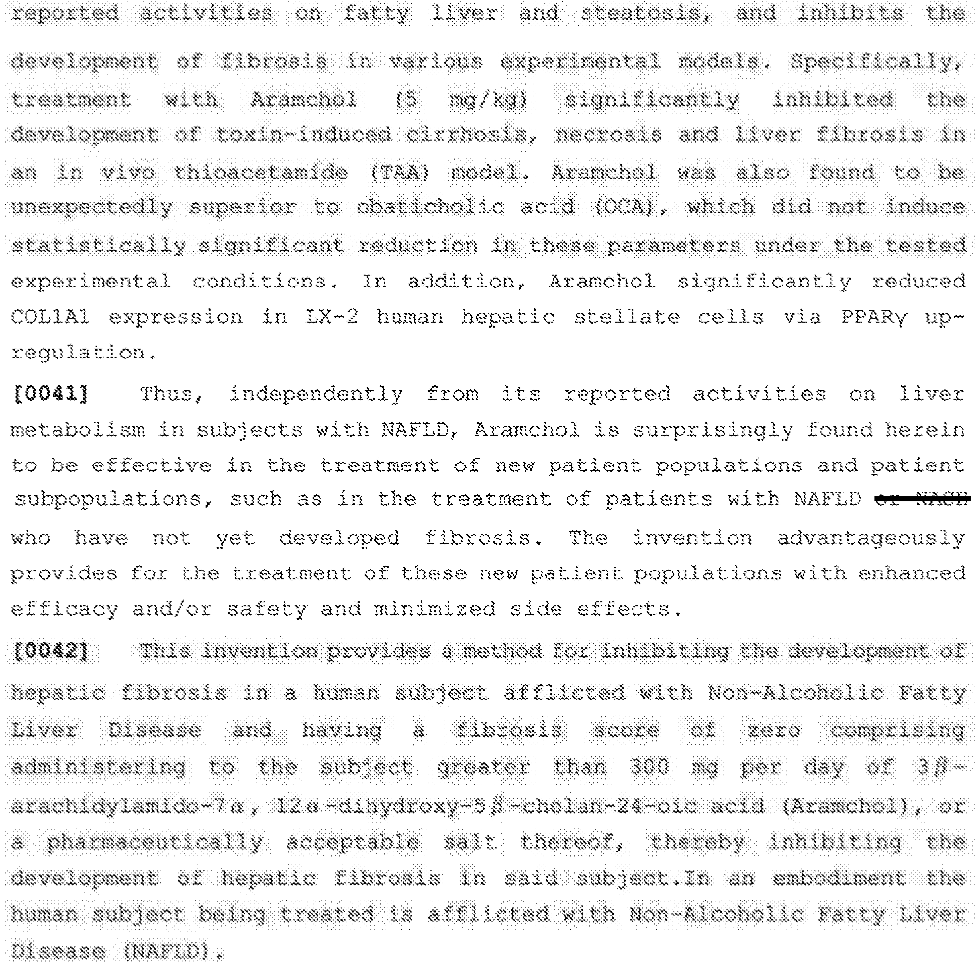

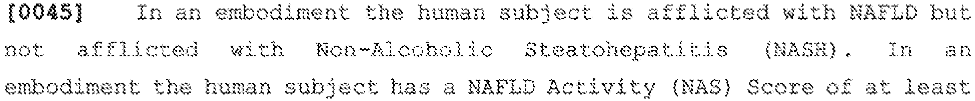

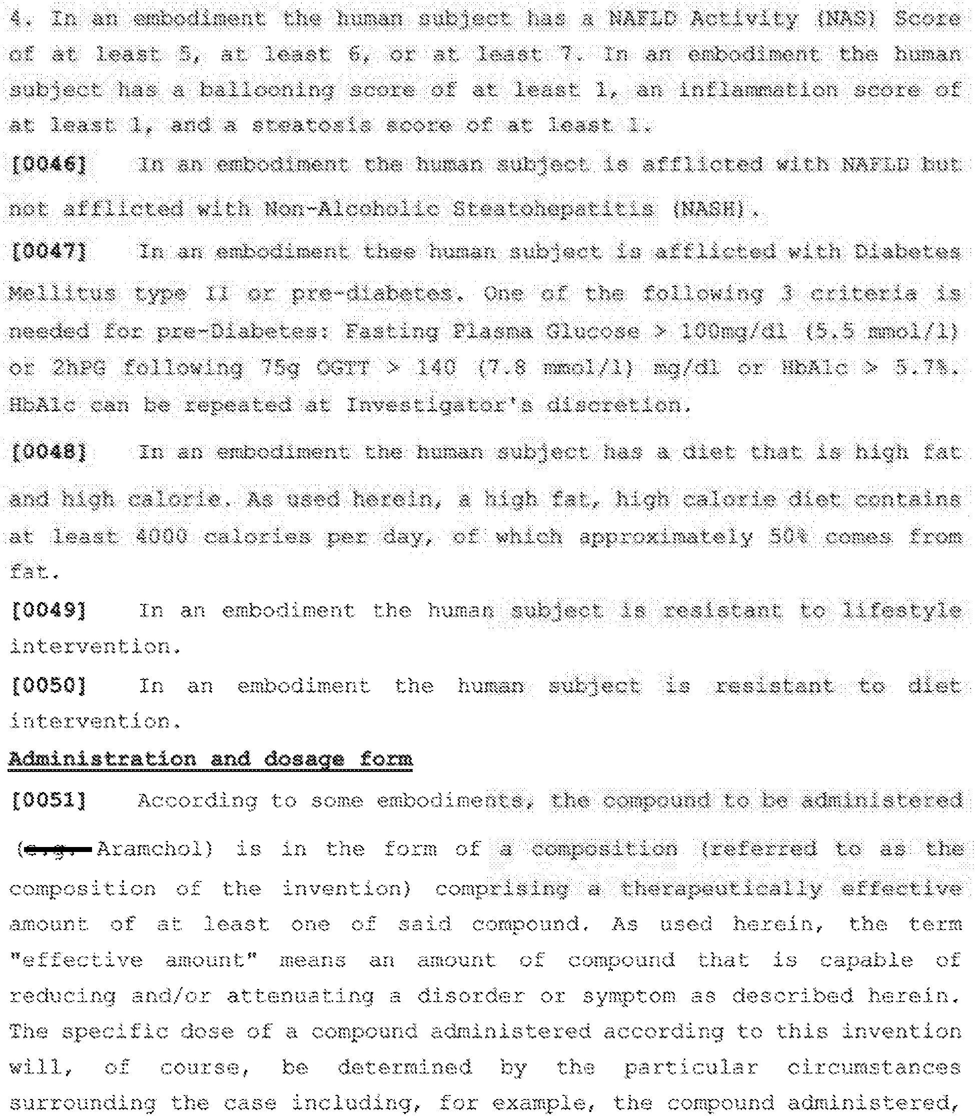

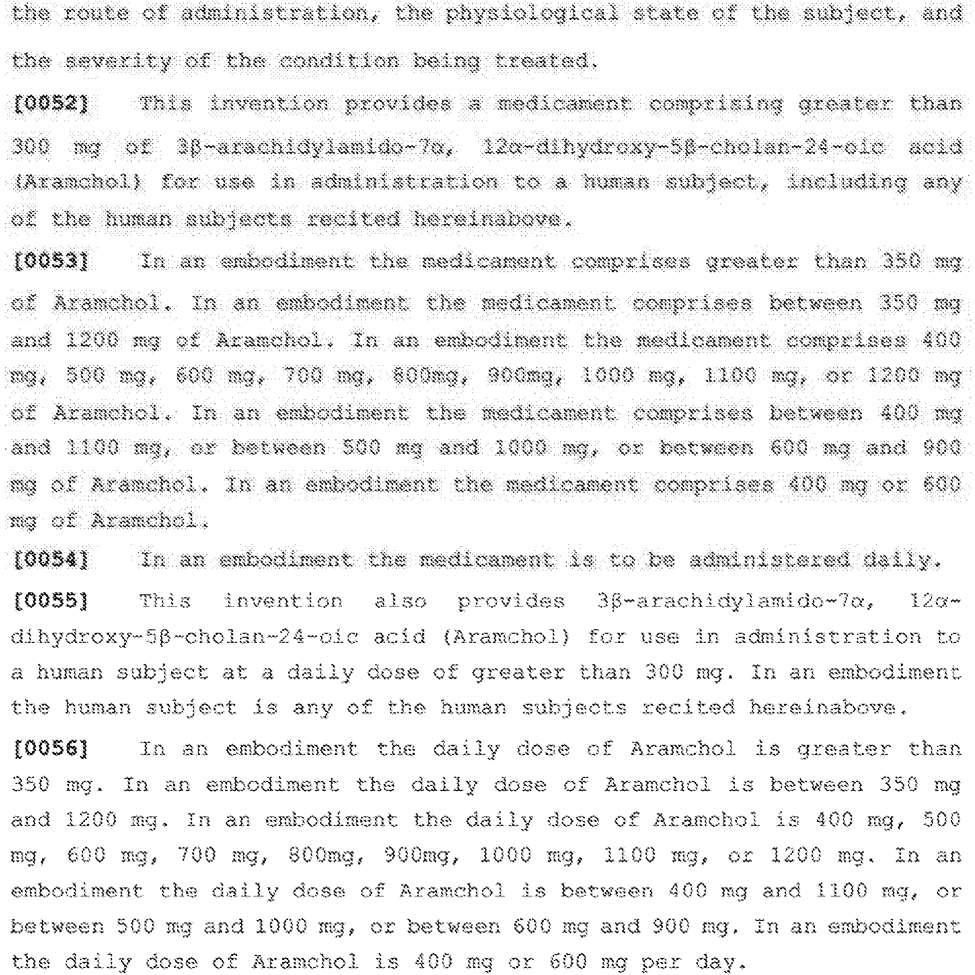

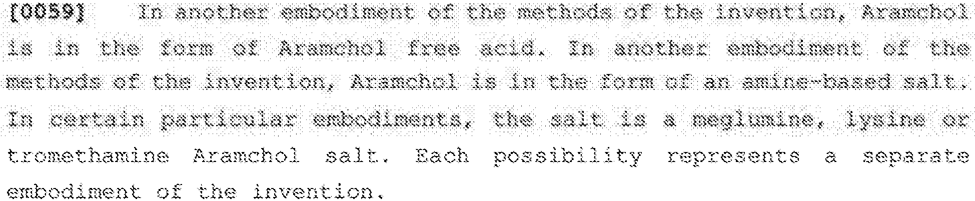

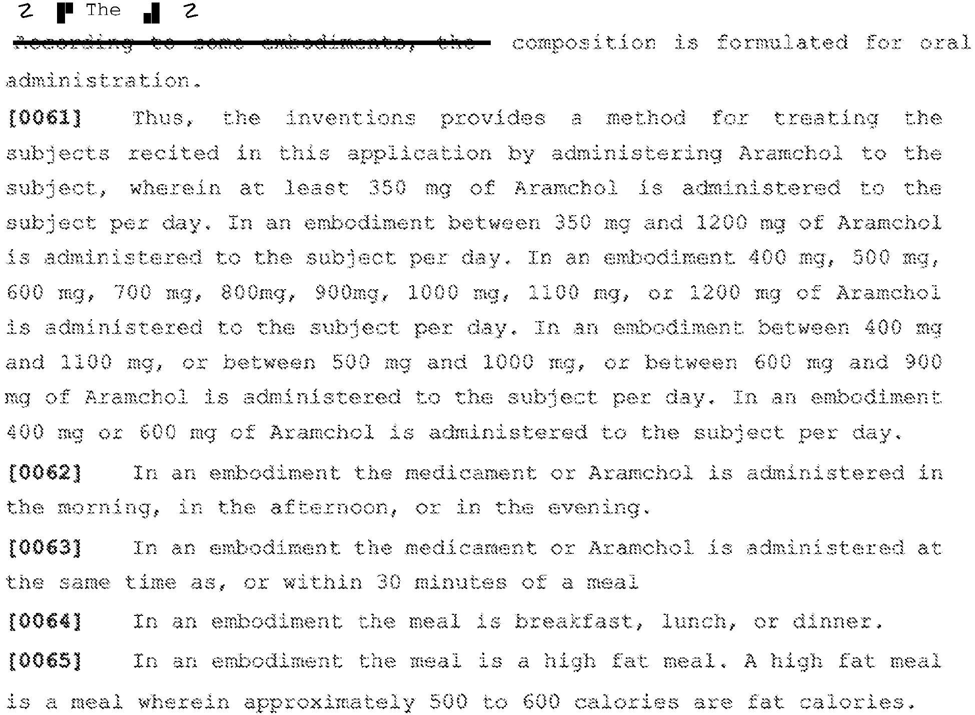

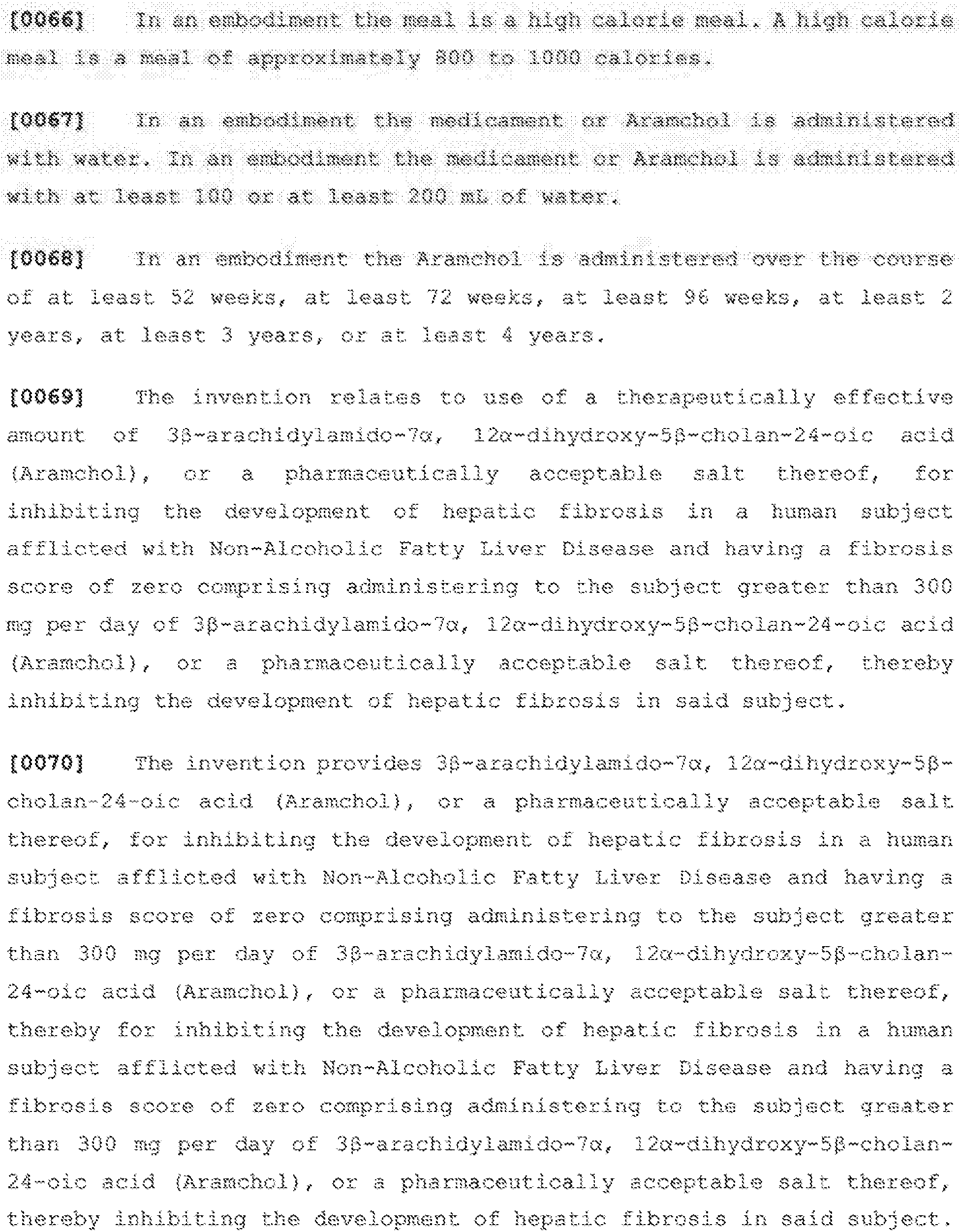

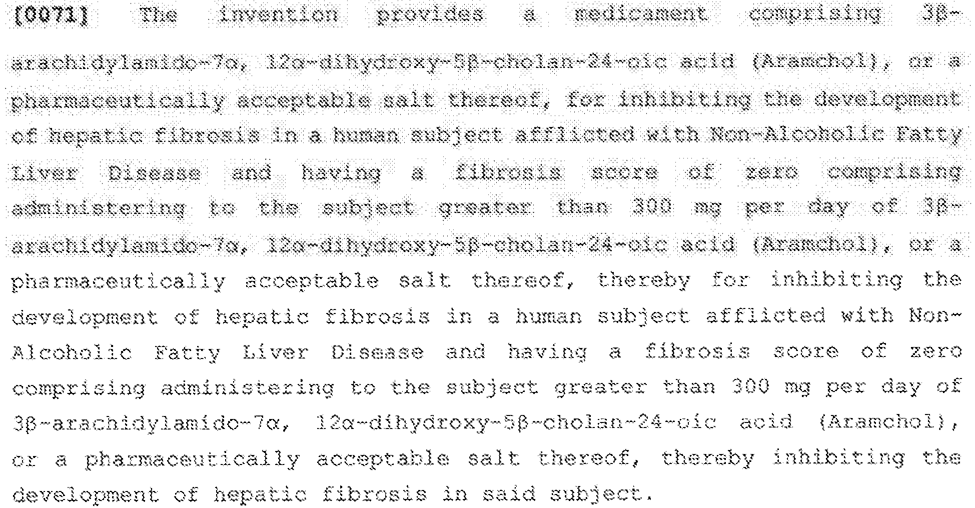

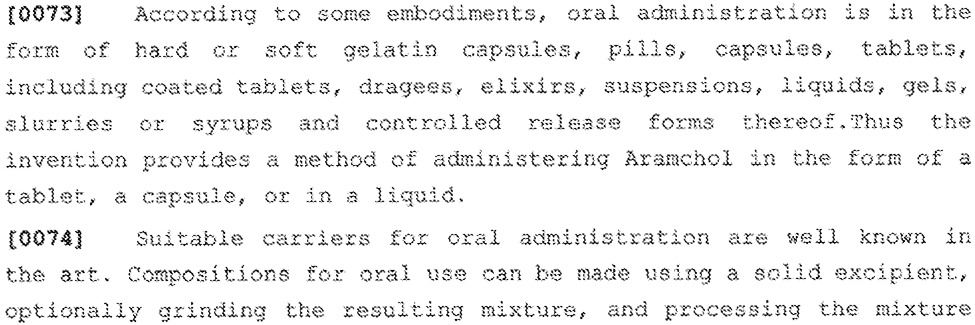

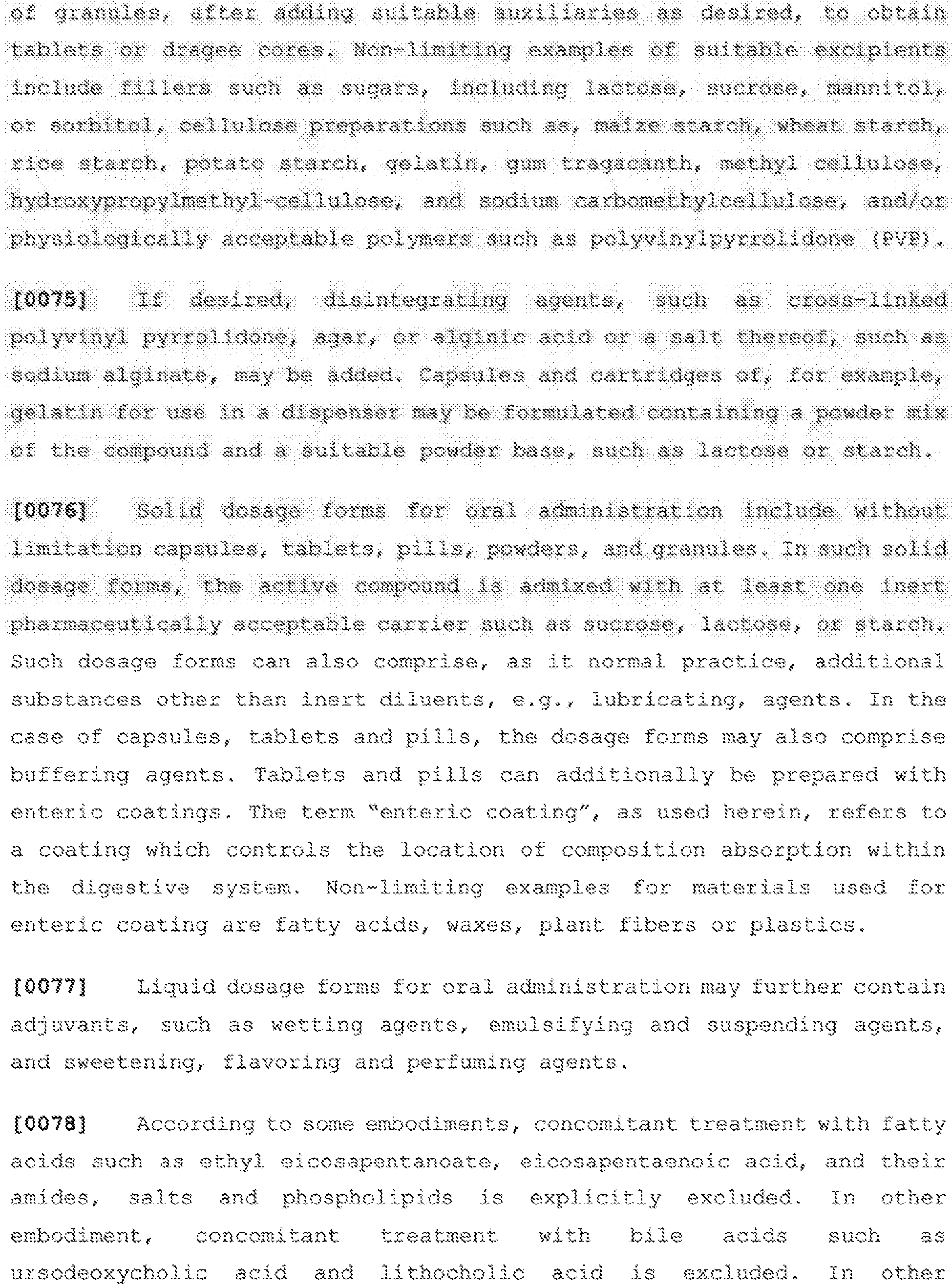

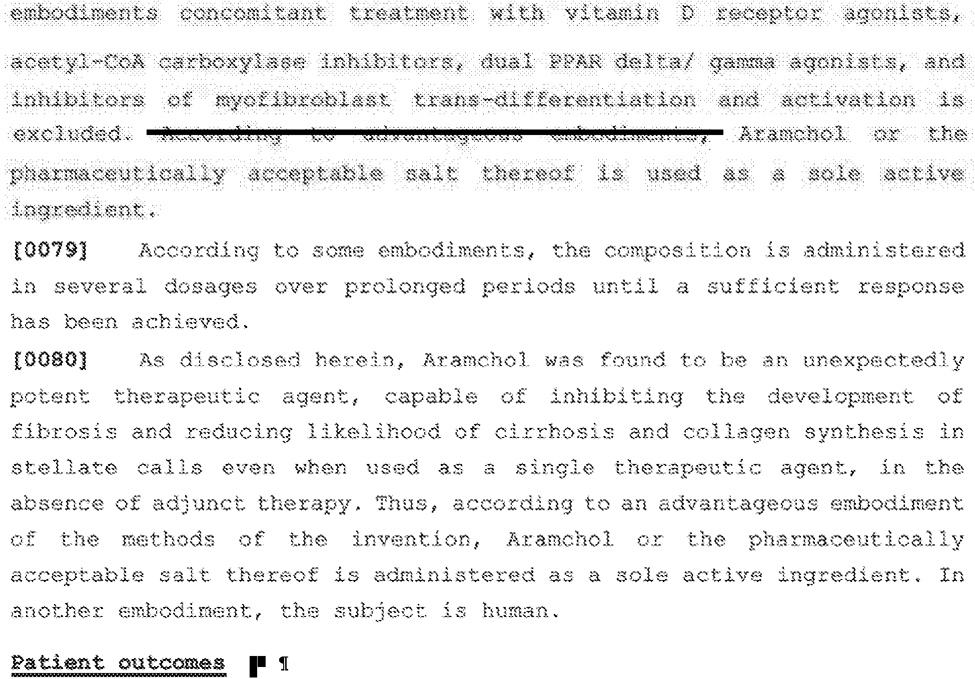

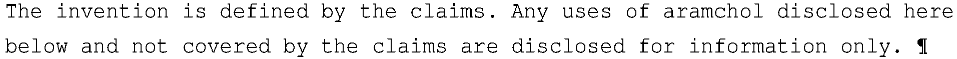

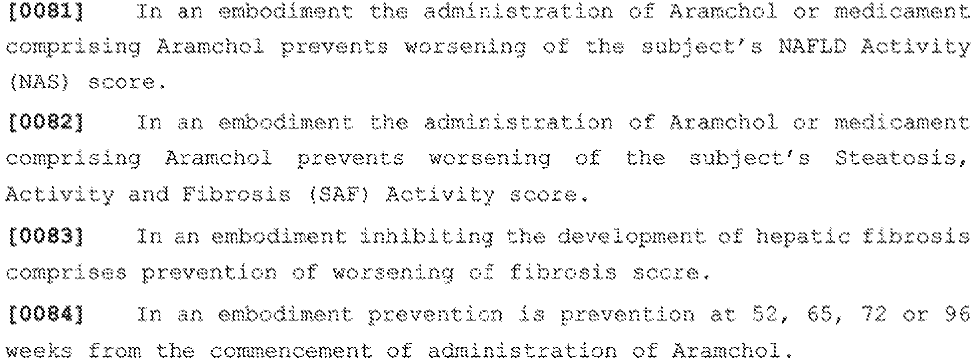

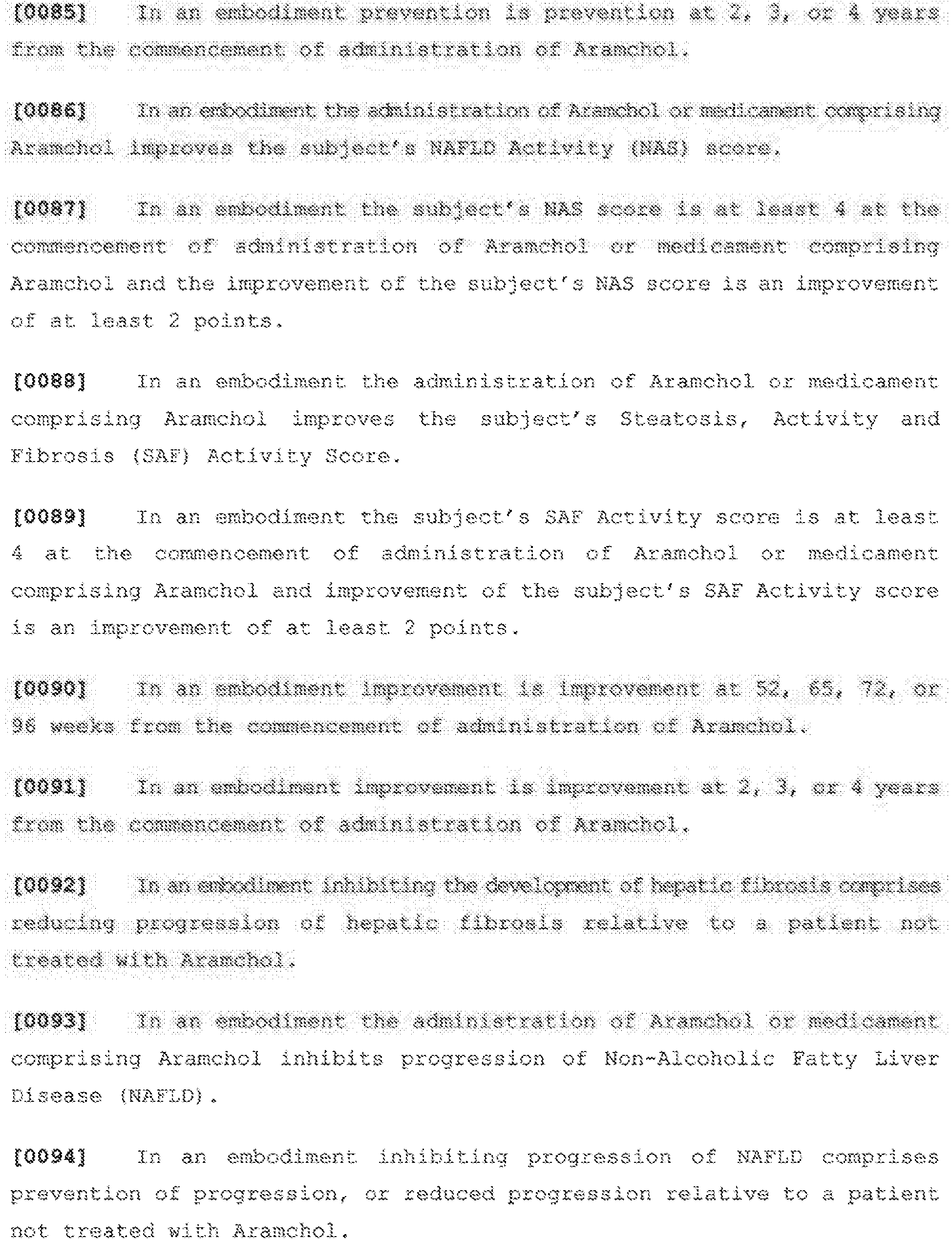

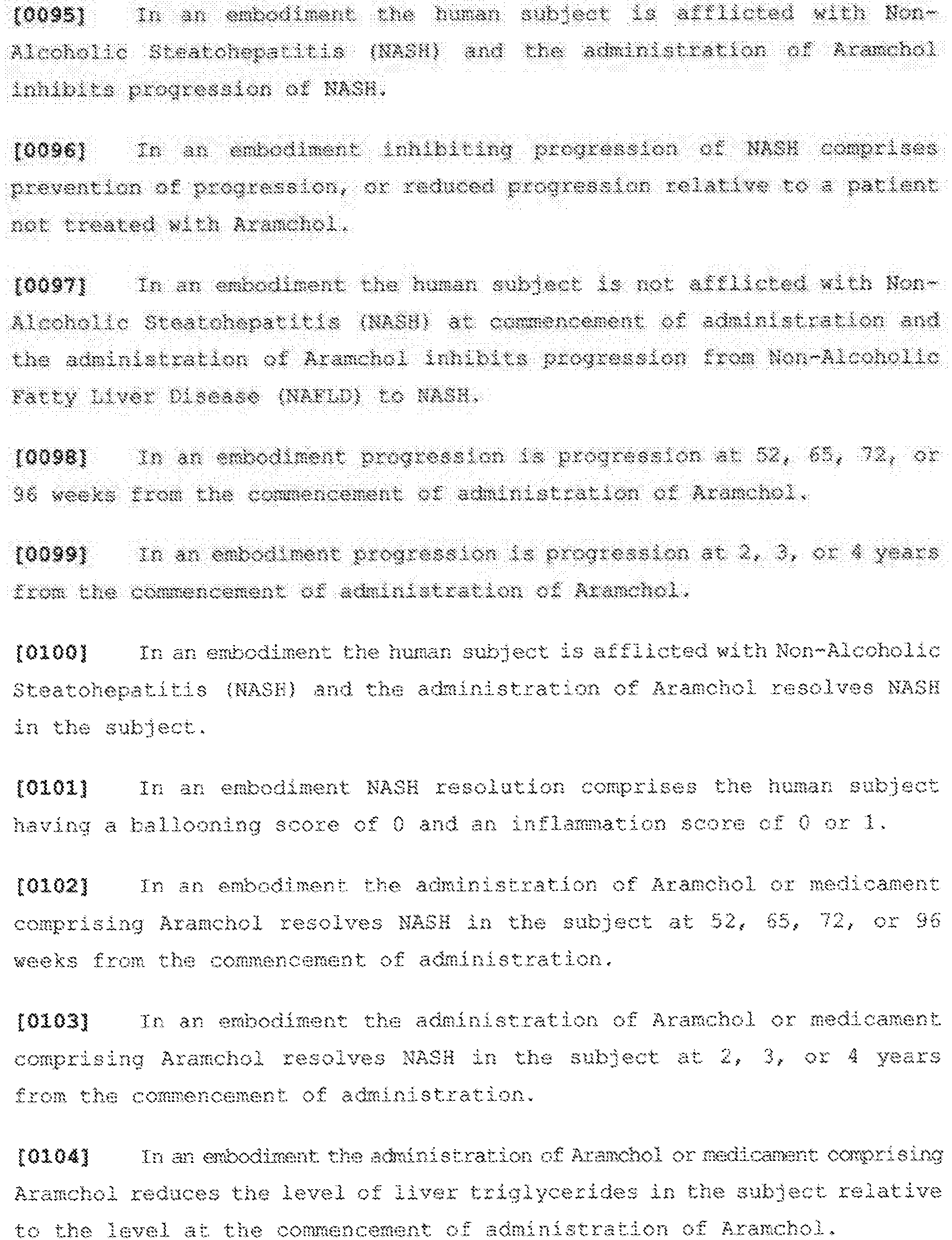

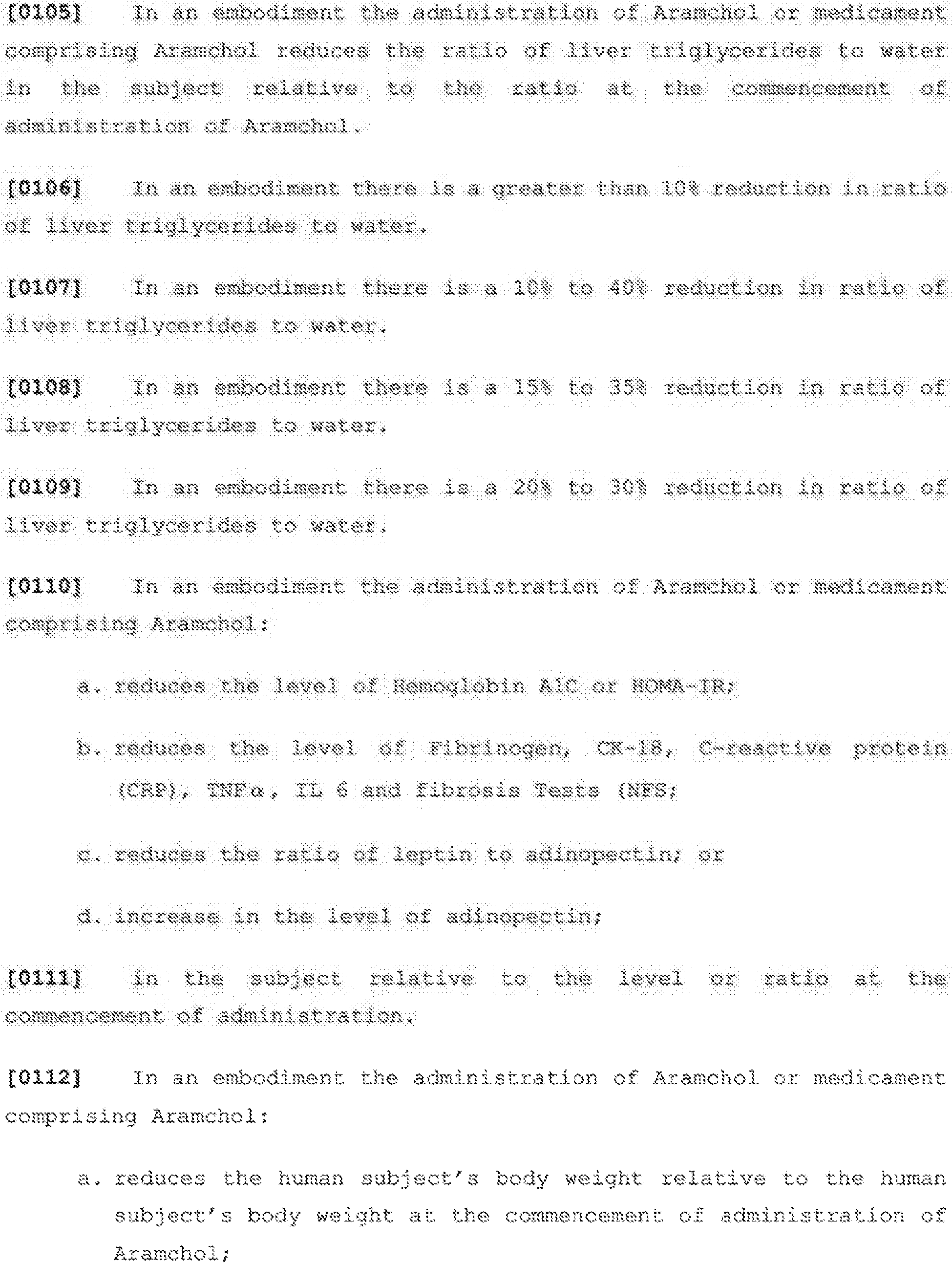

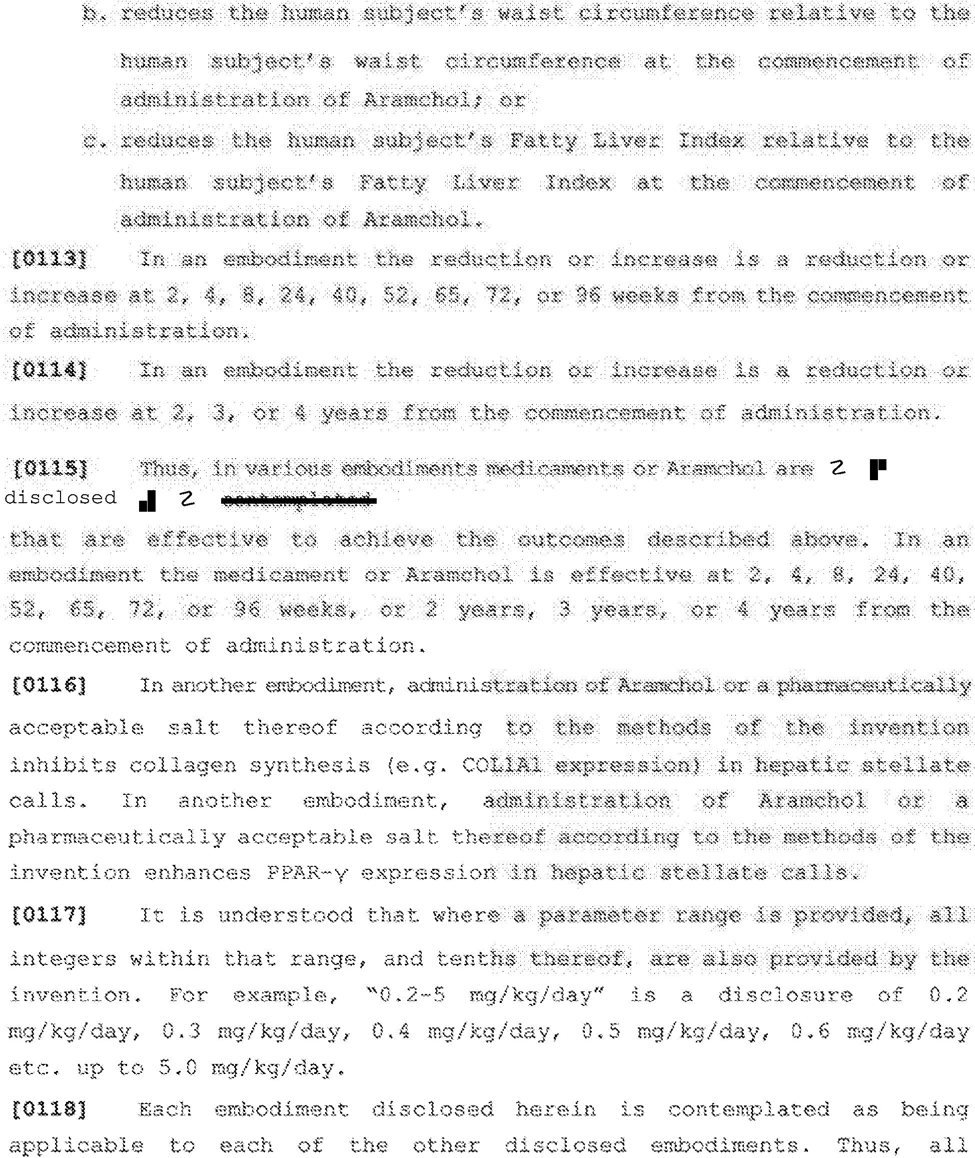

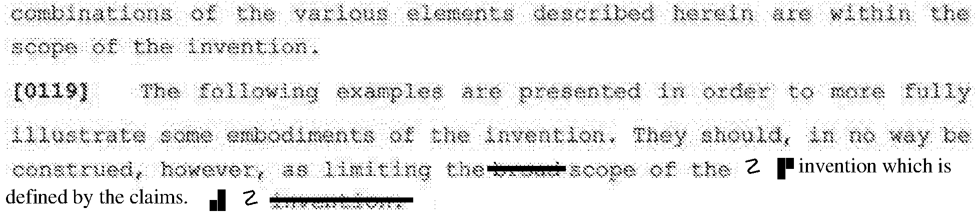

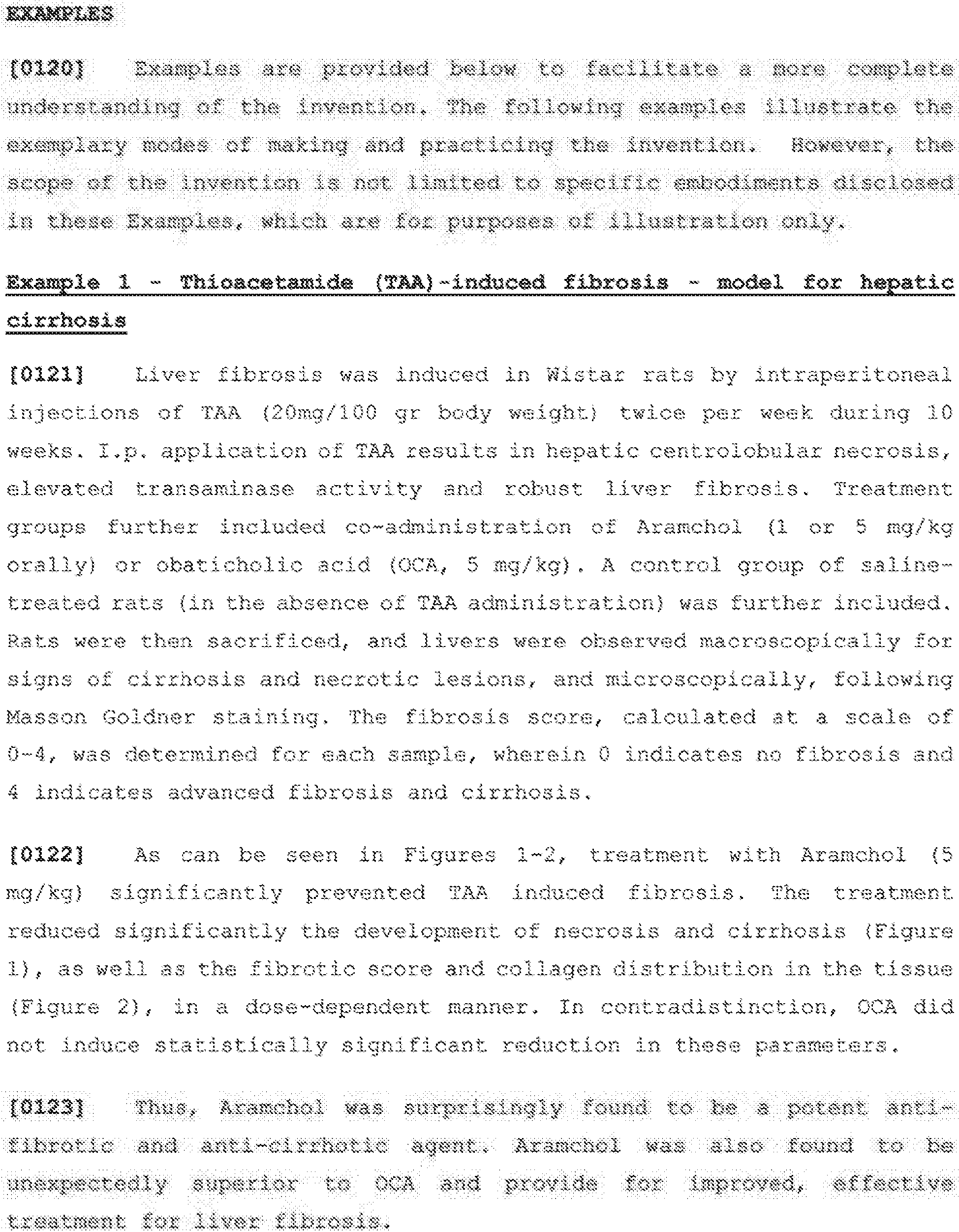

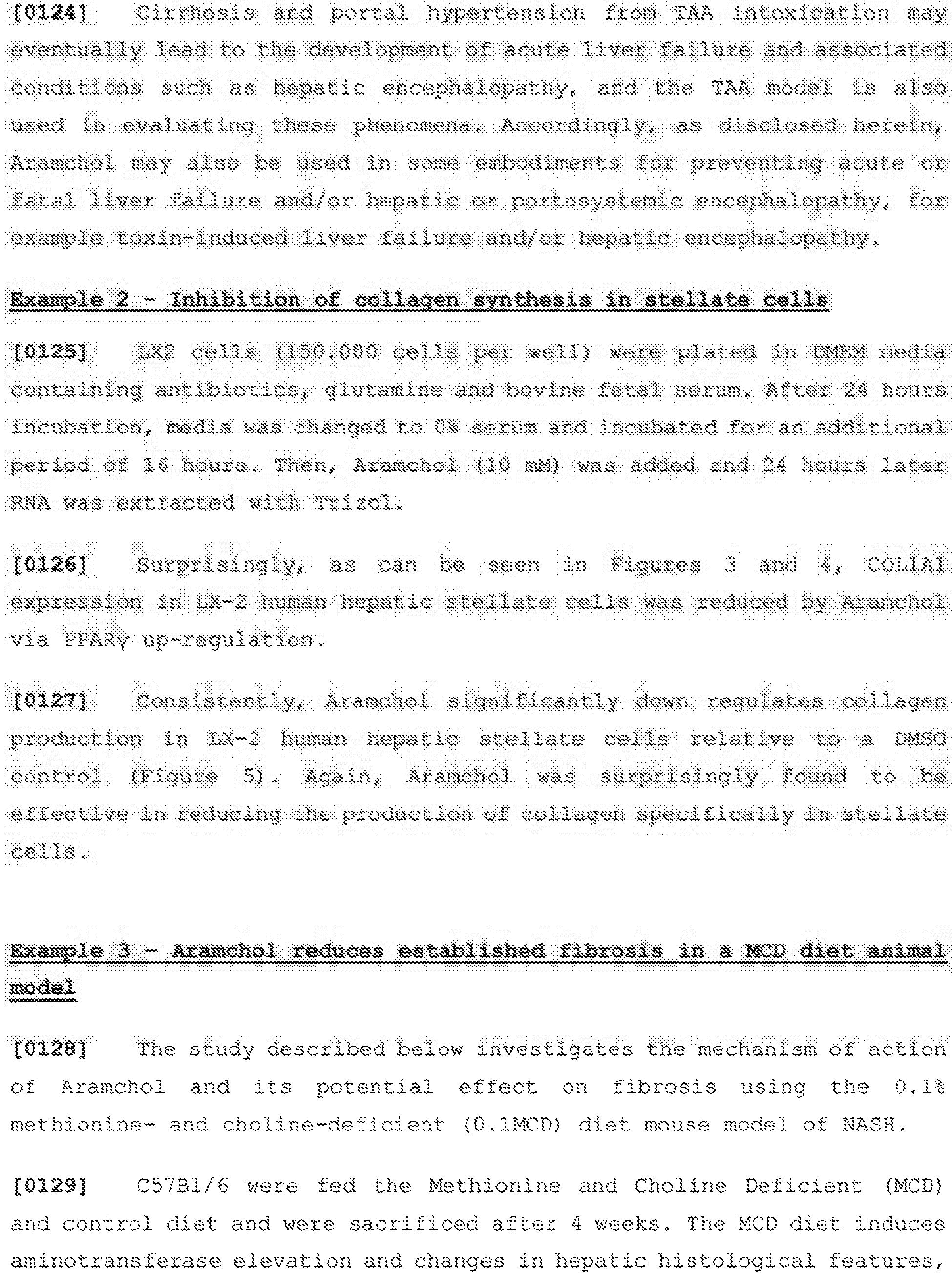

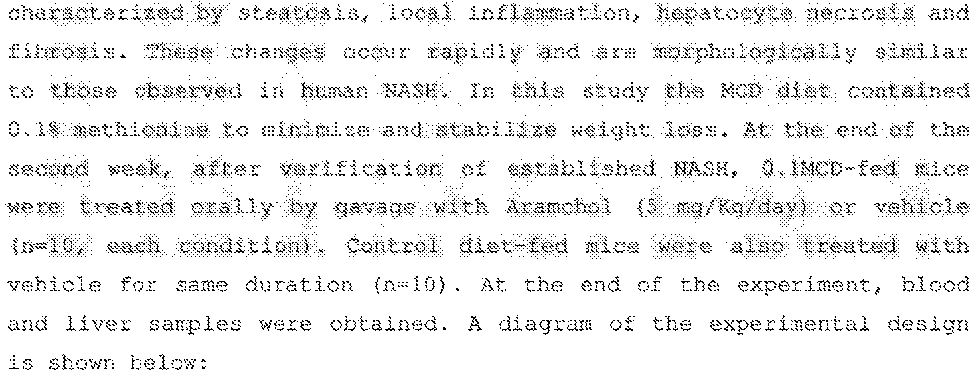

| | |
|---|---|
| 0.1MCD diet | 0.1MCD diet + Aramchol^{™} 5mg/kg |
| 14 days | 14 days |
| 28 days | |

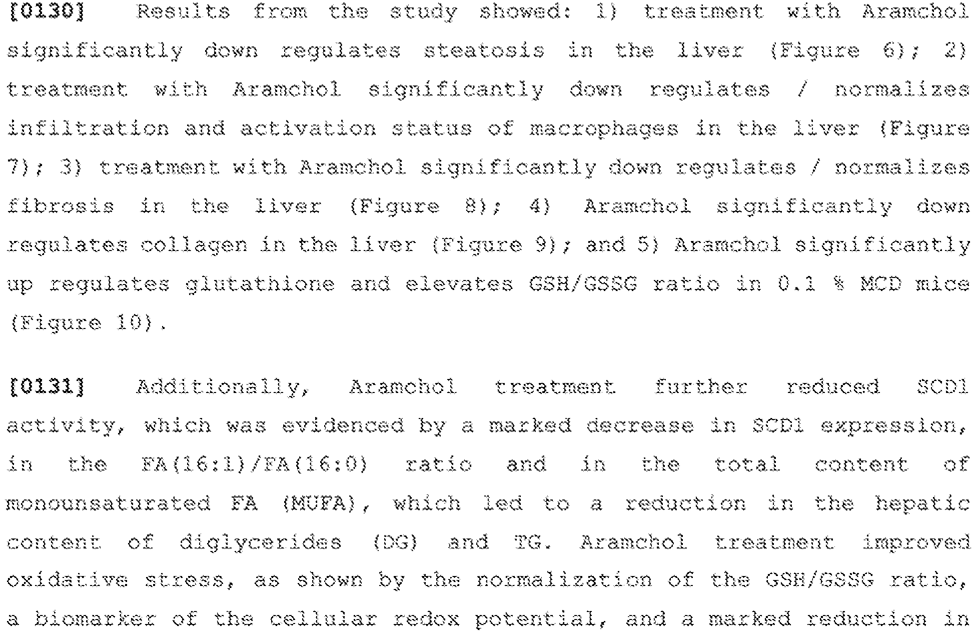

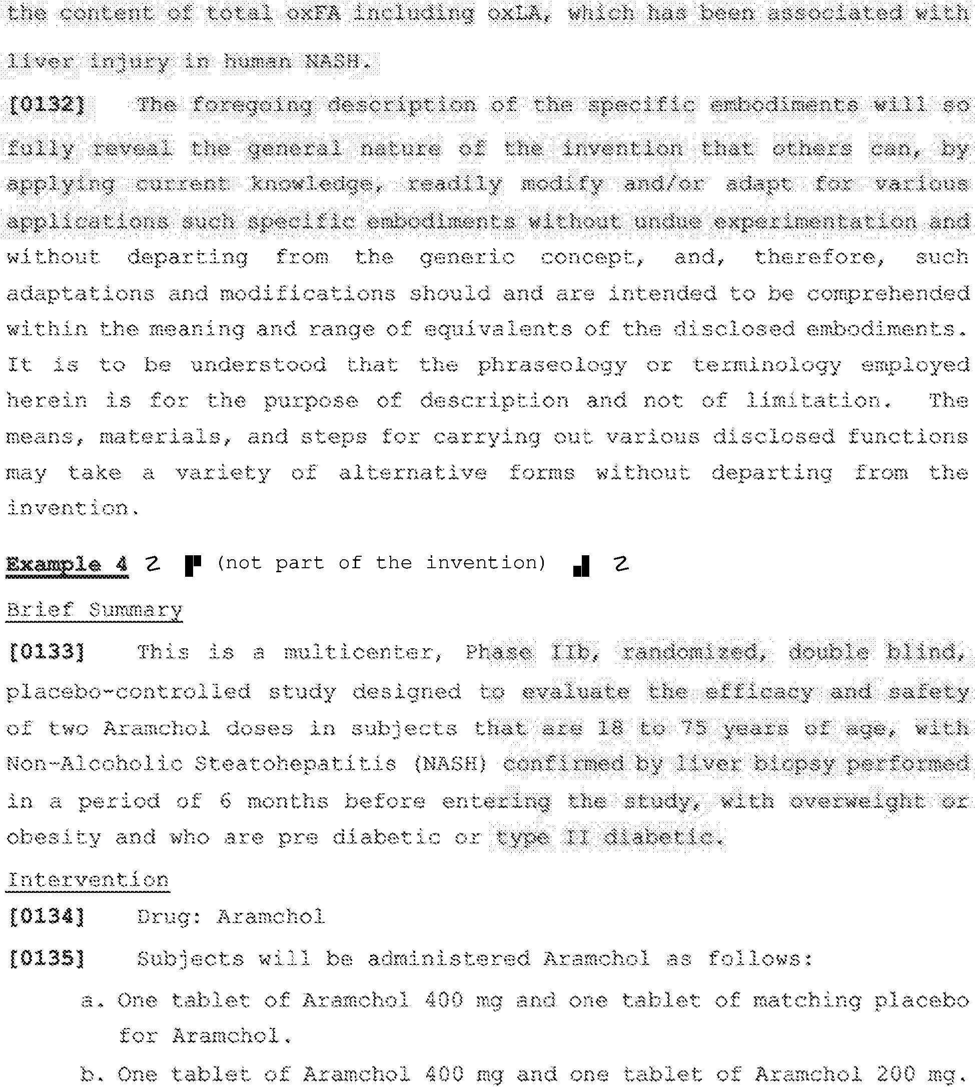

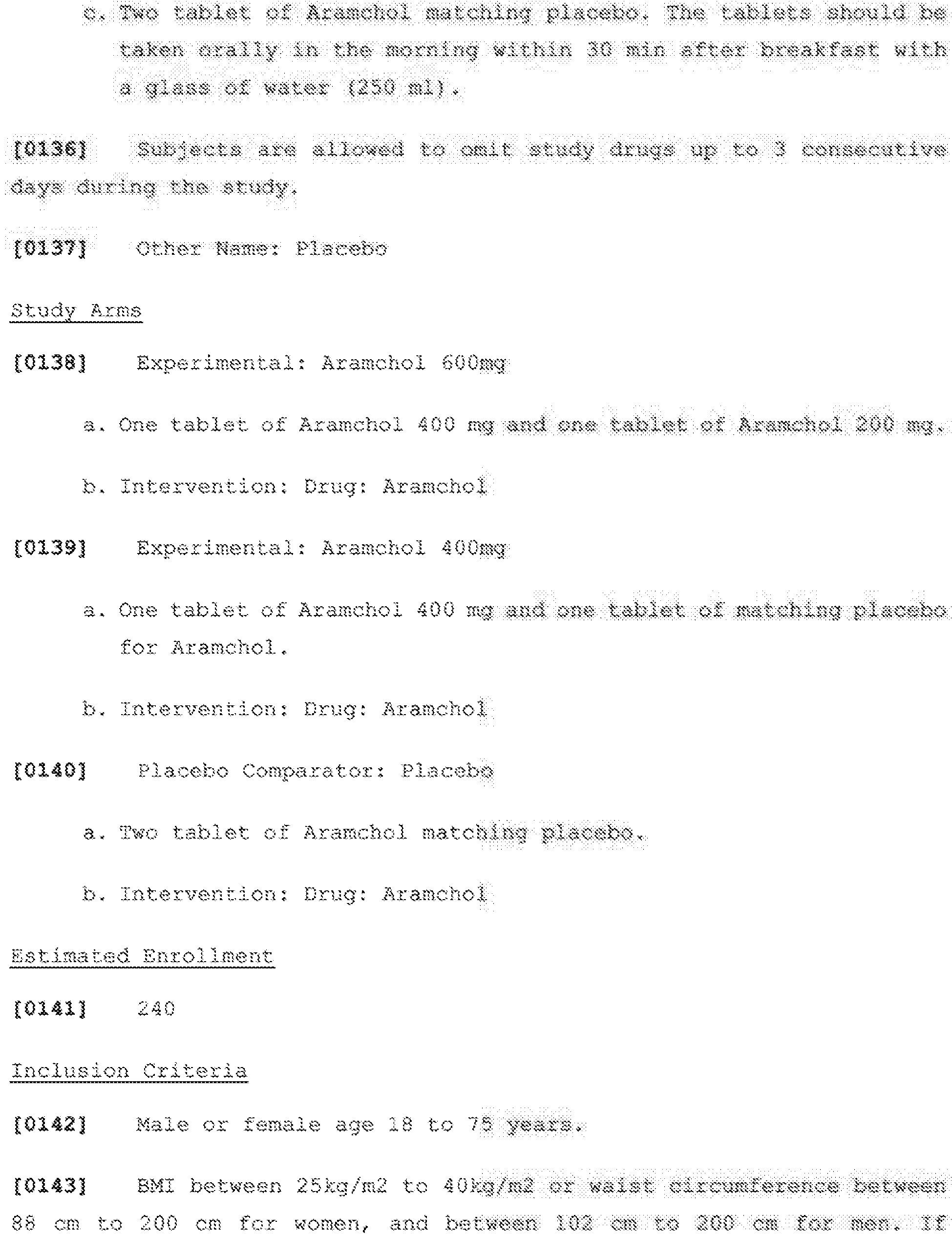

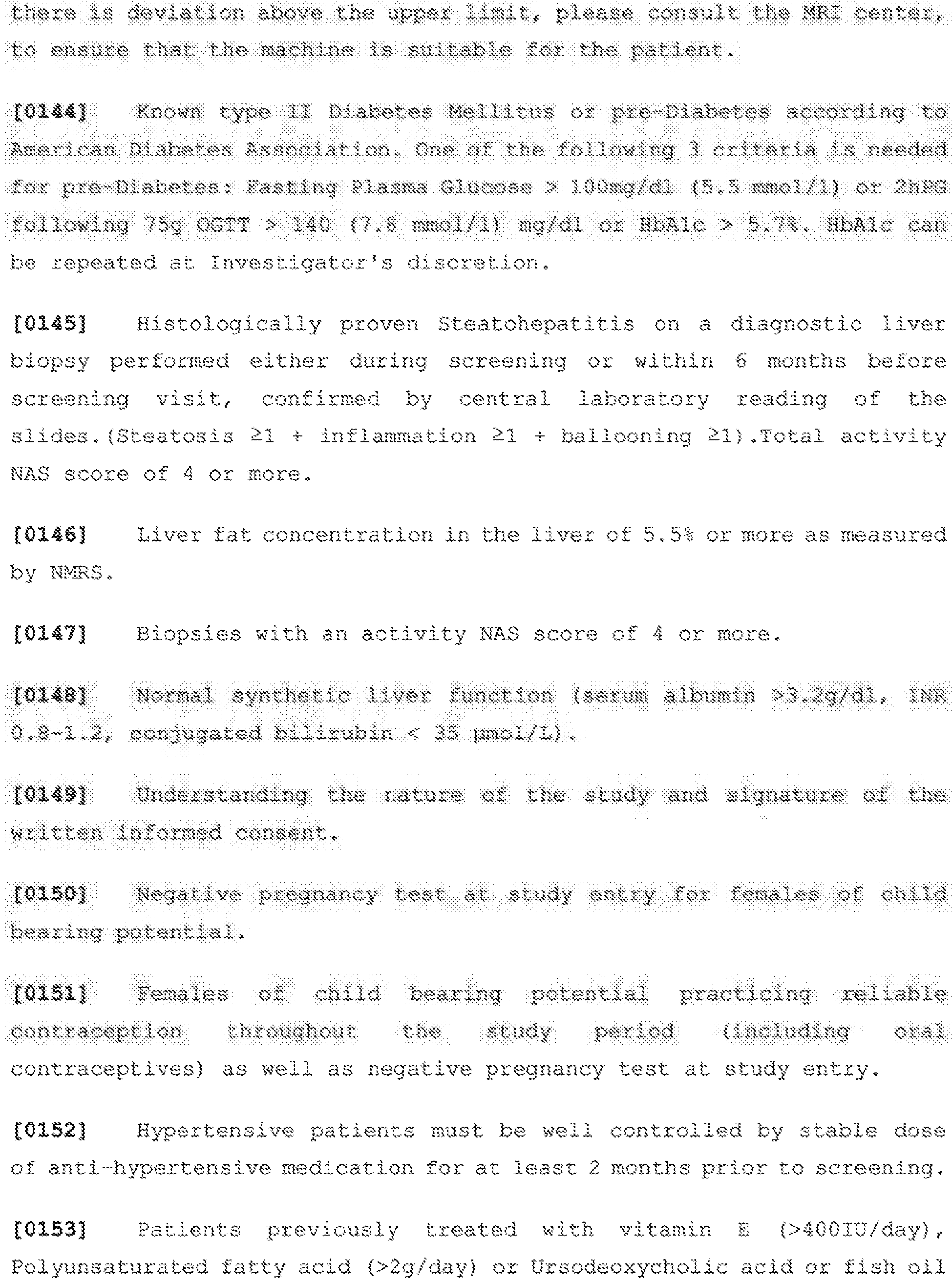

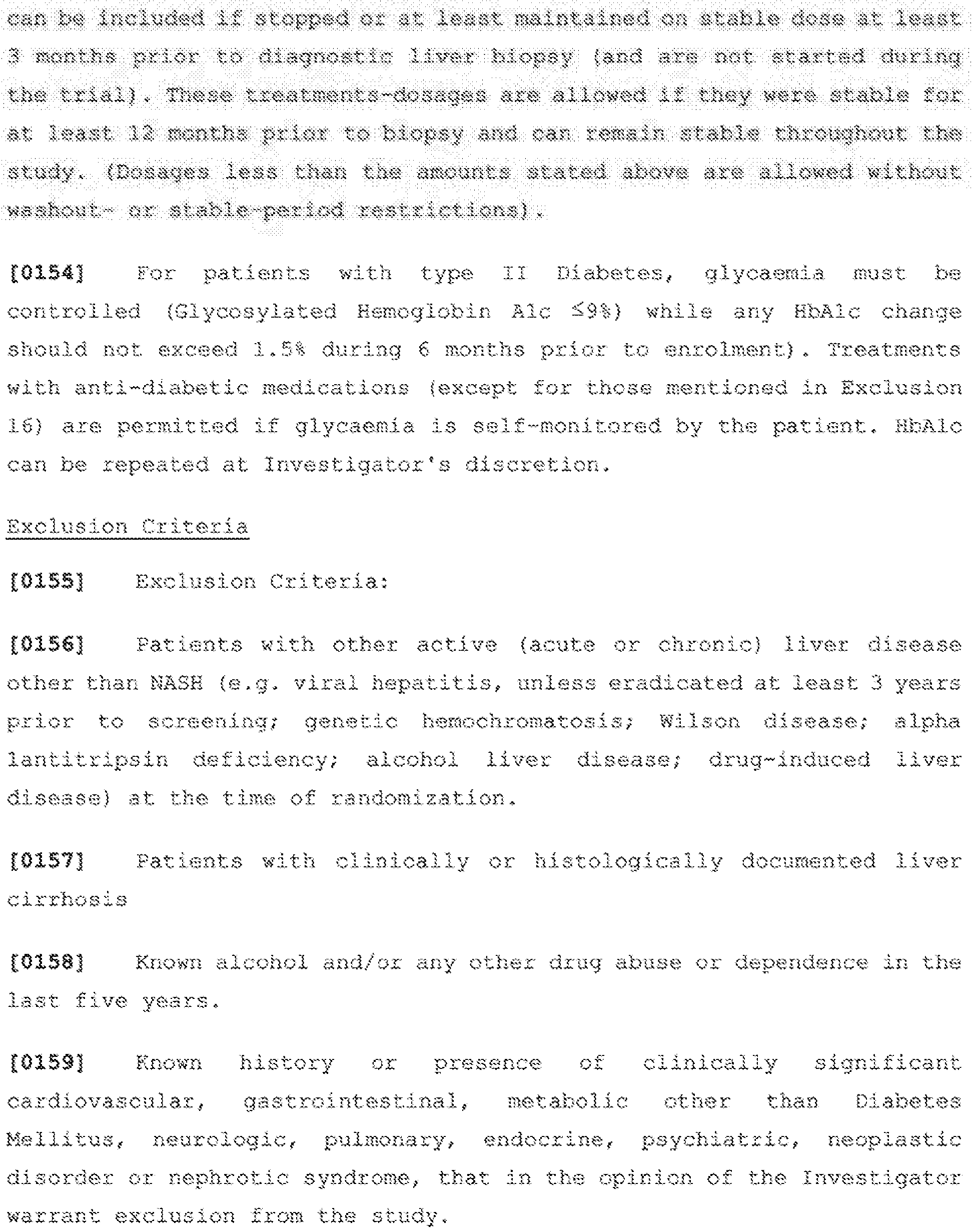

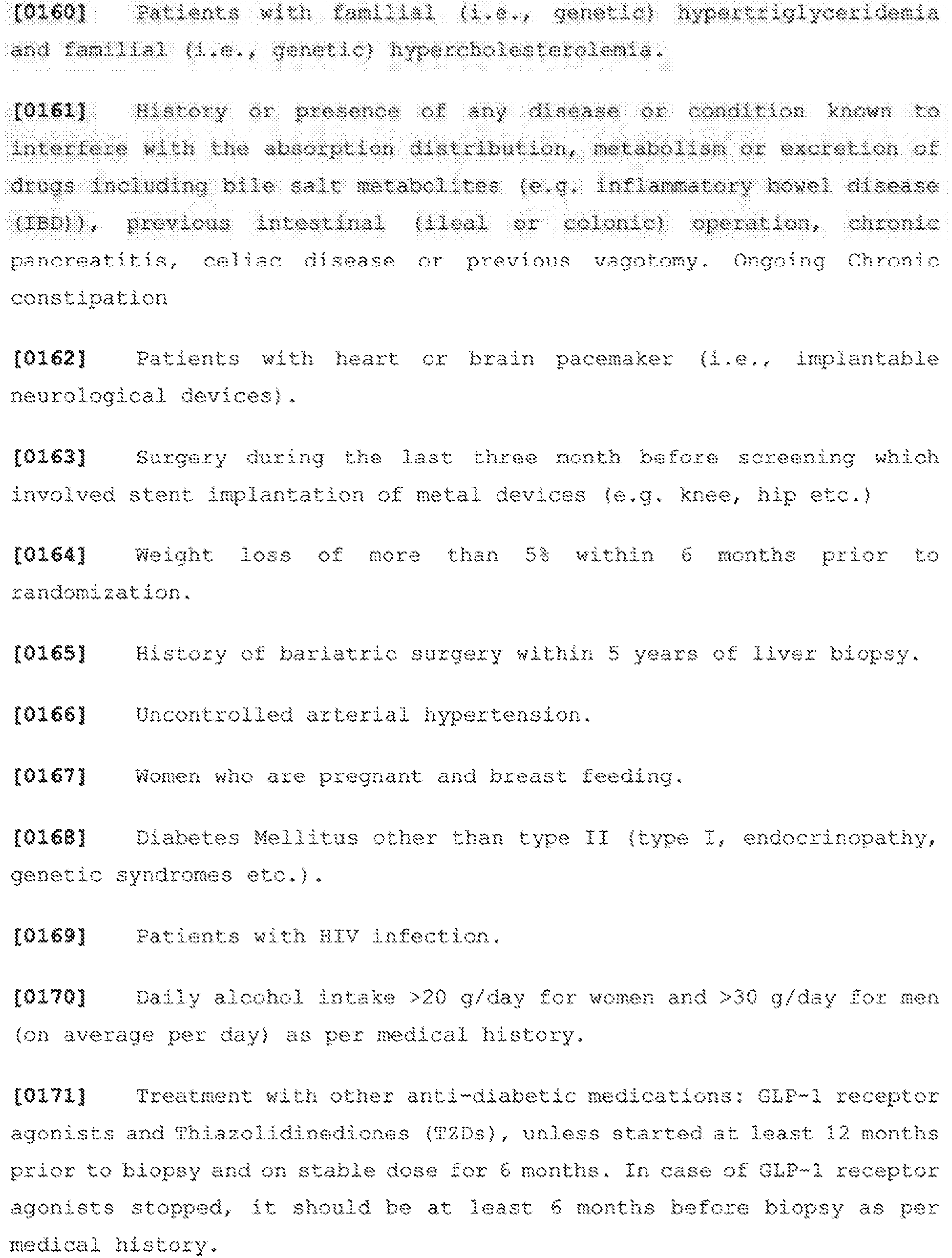

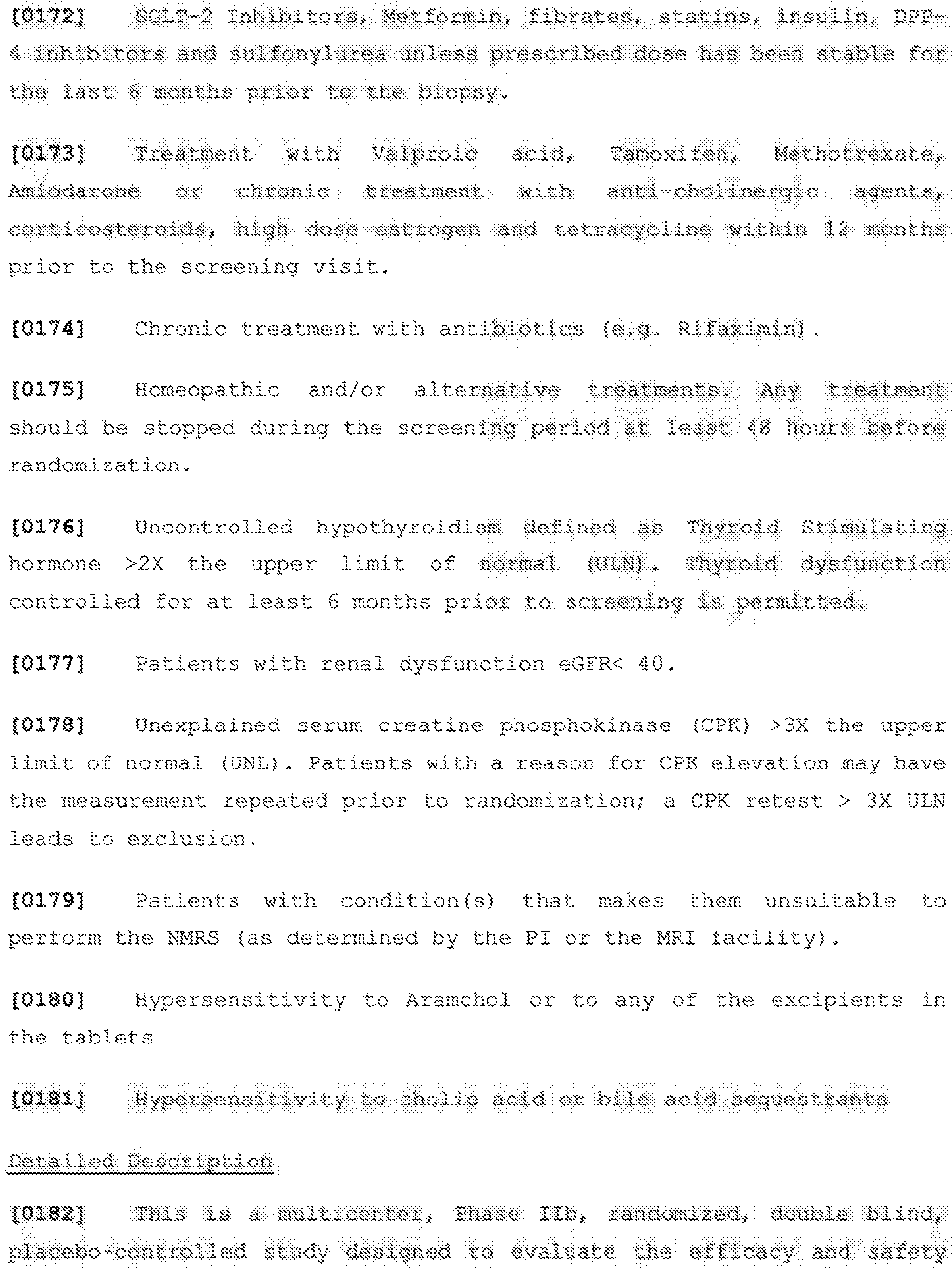

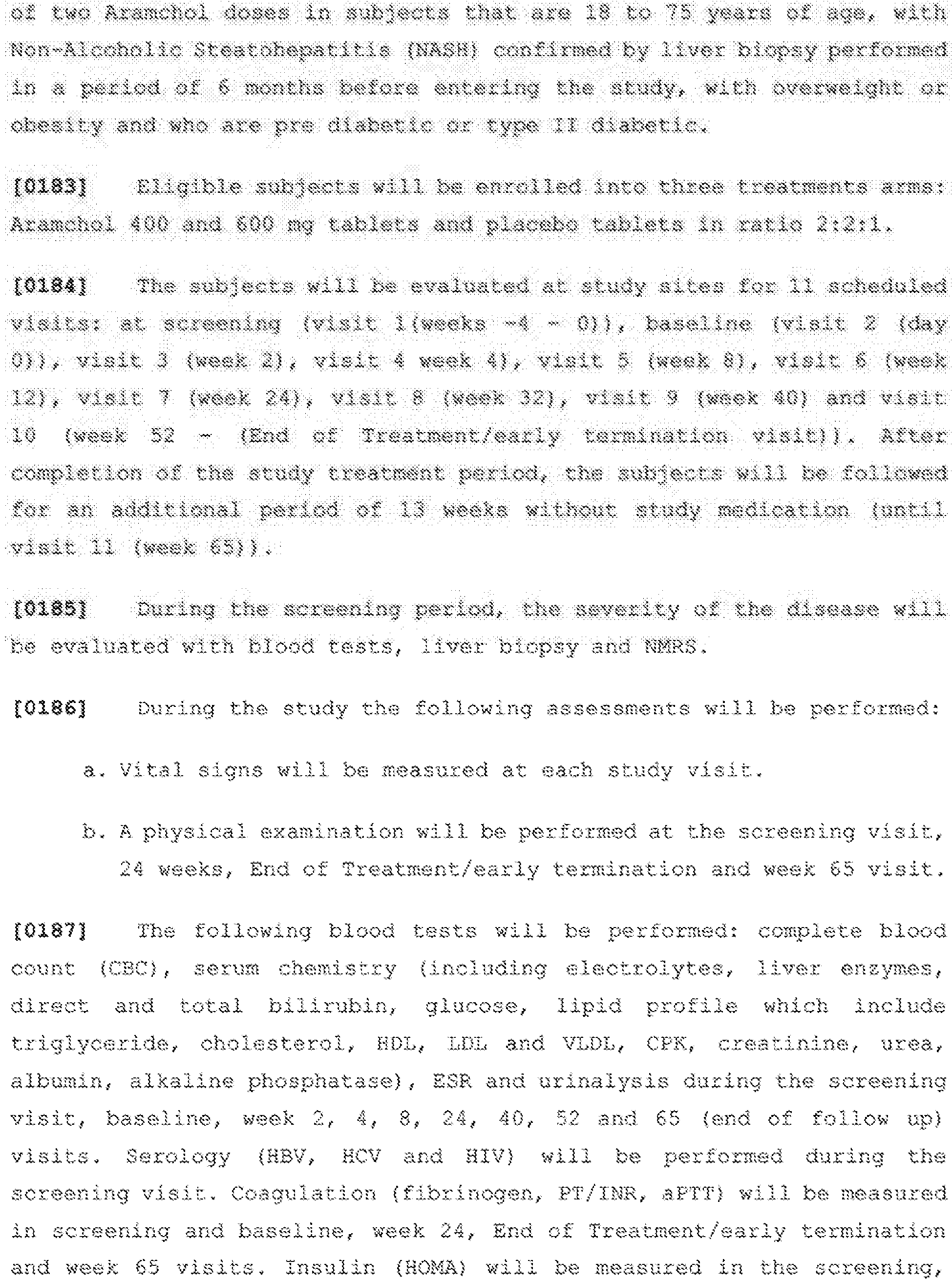

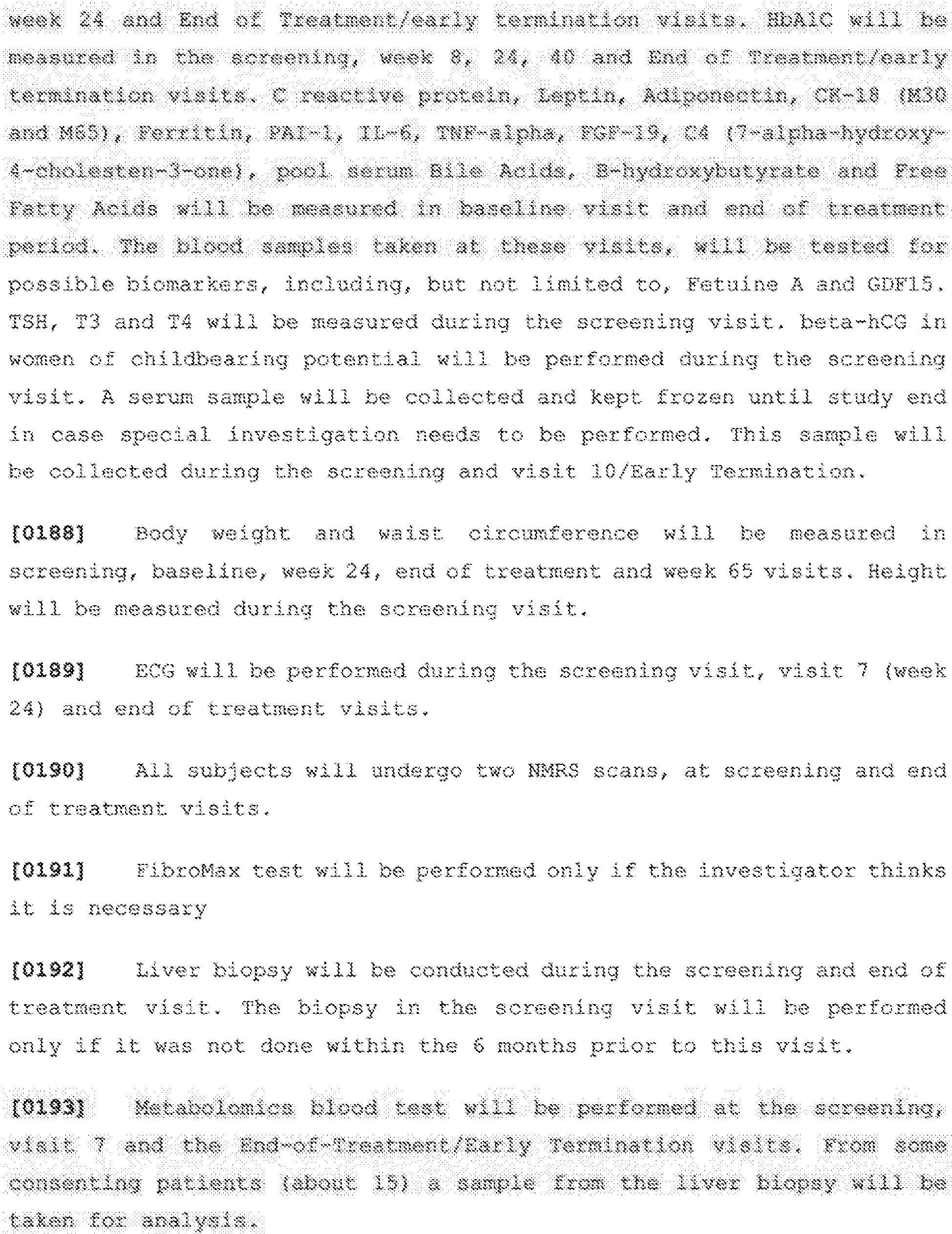

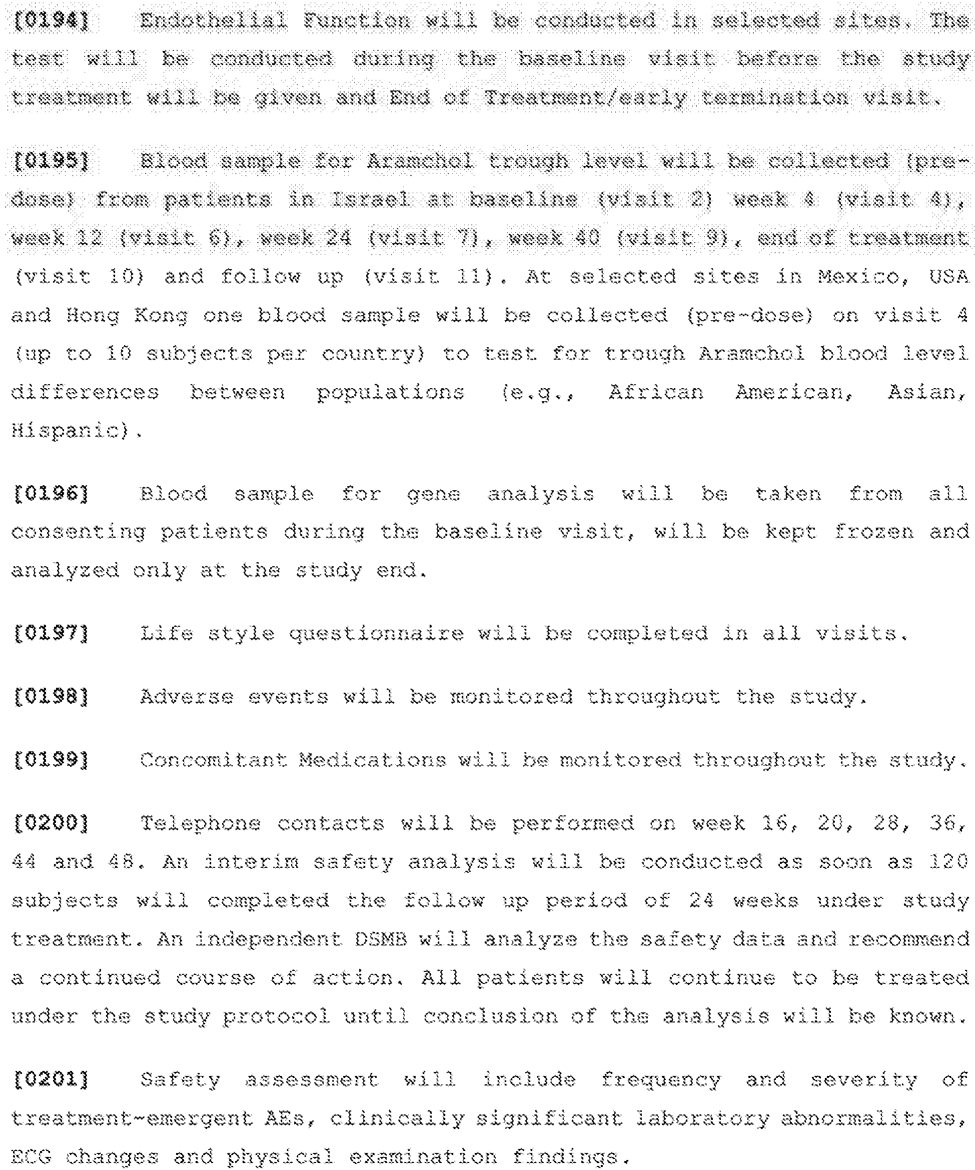

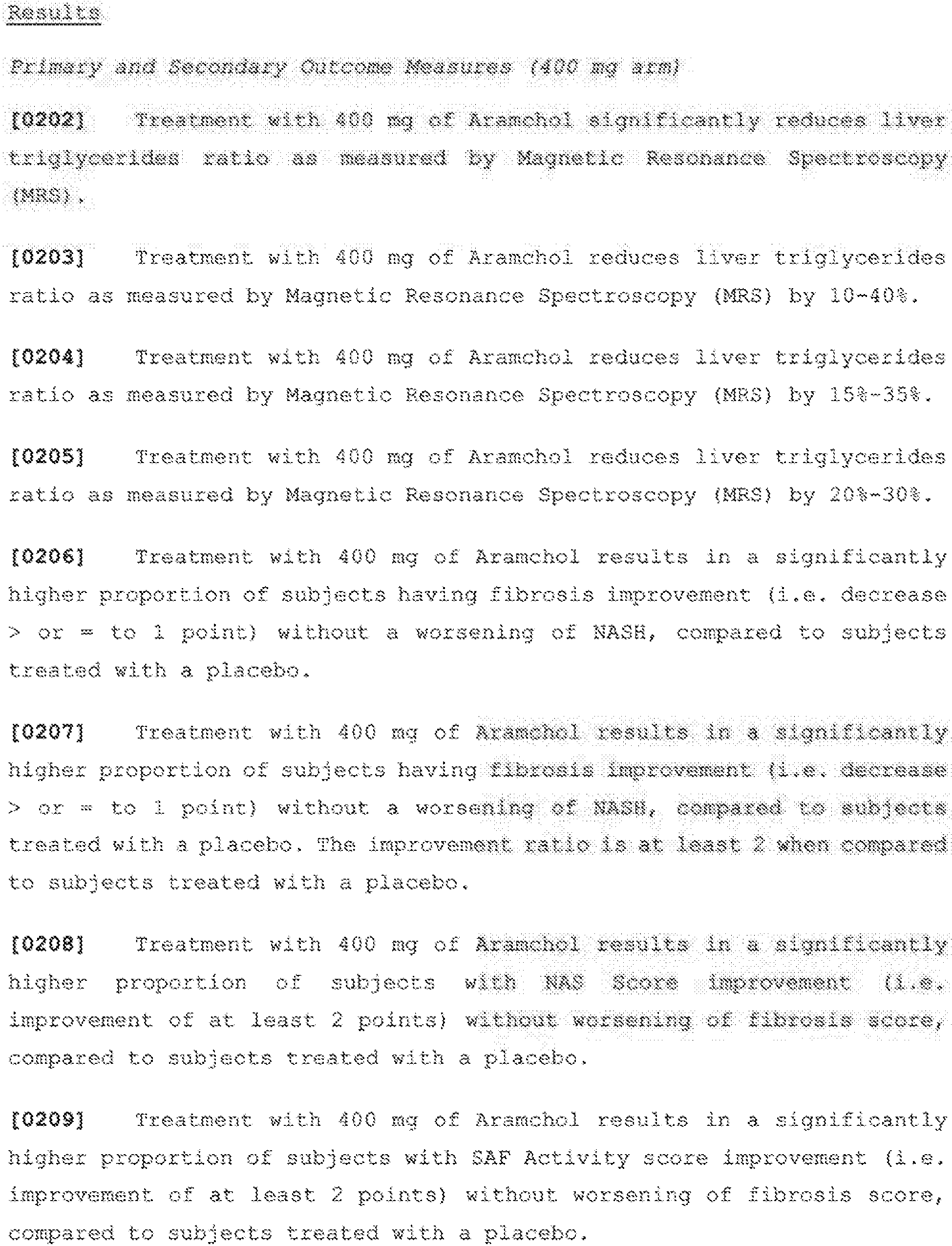

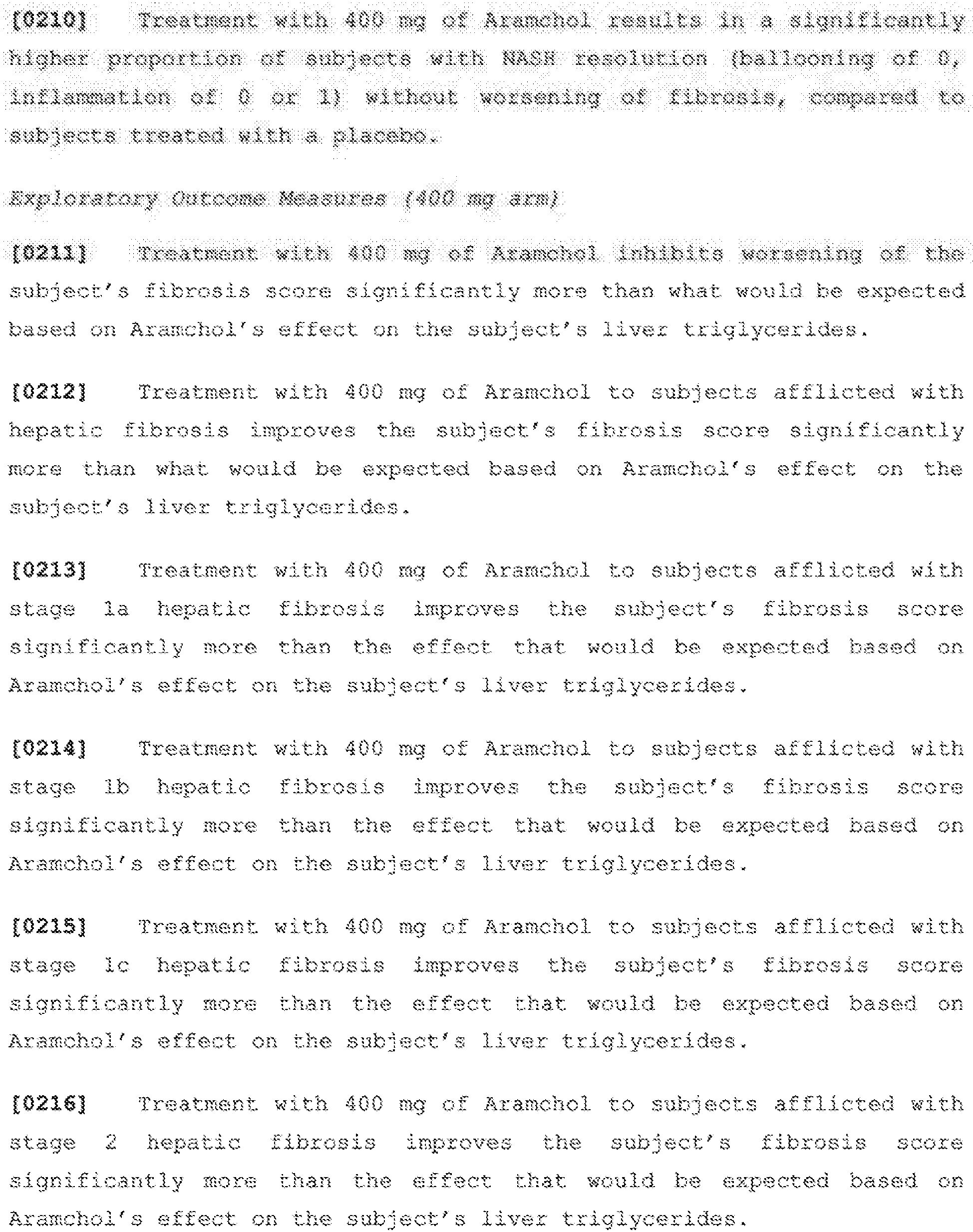

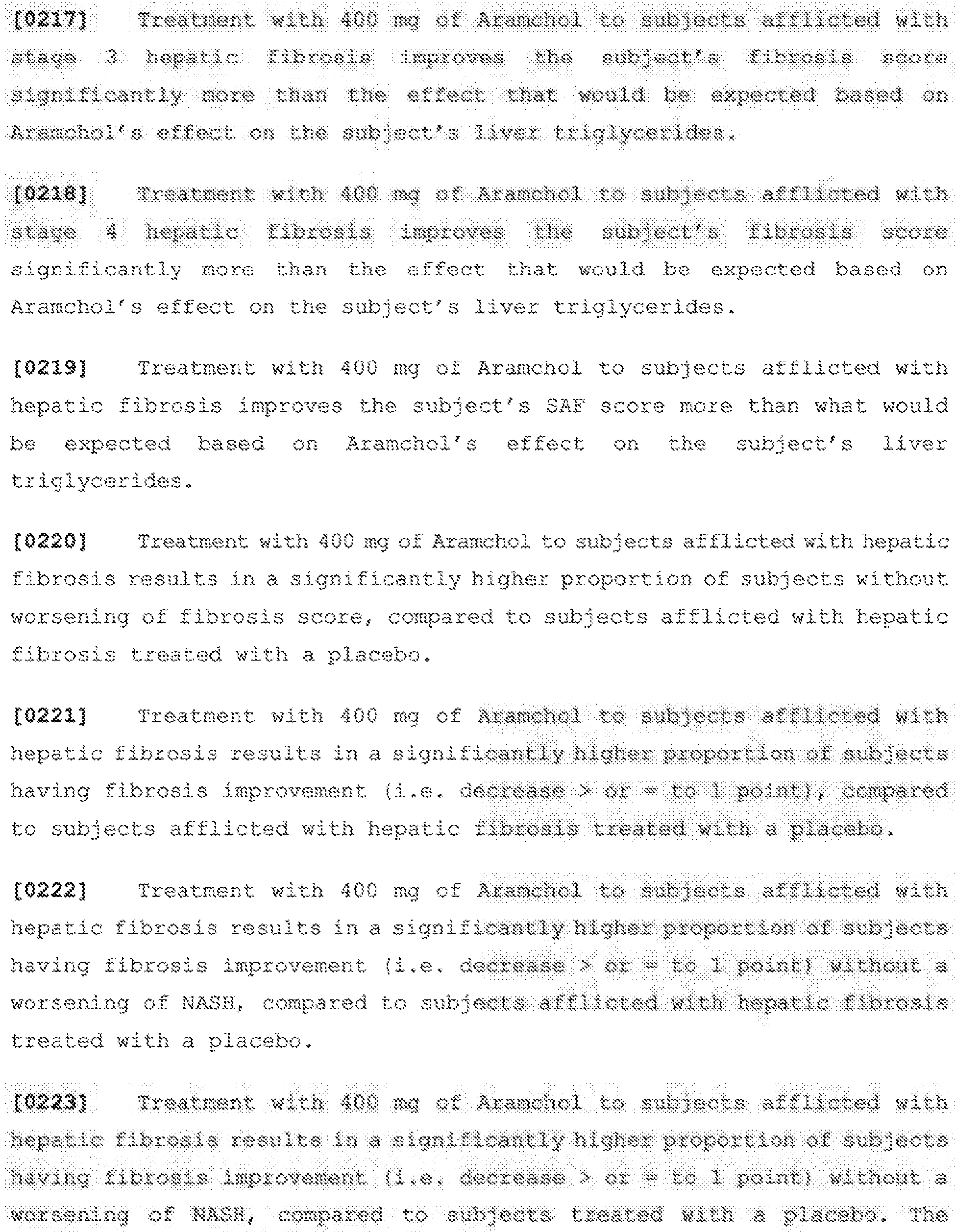

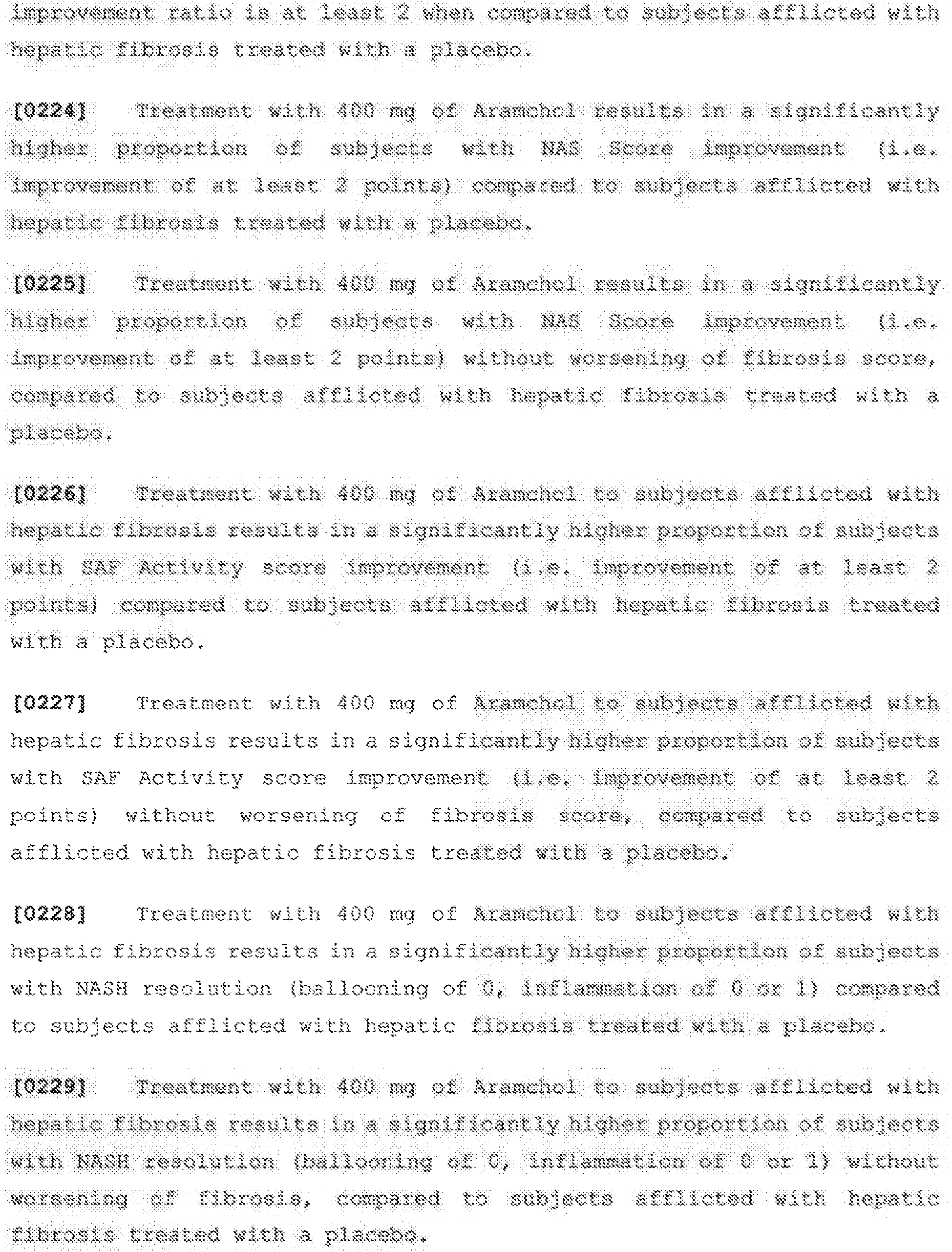

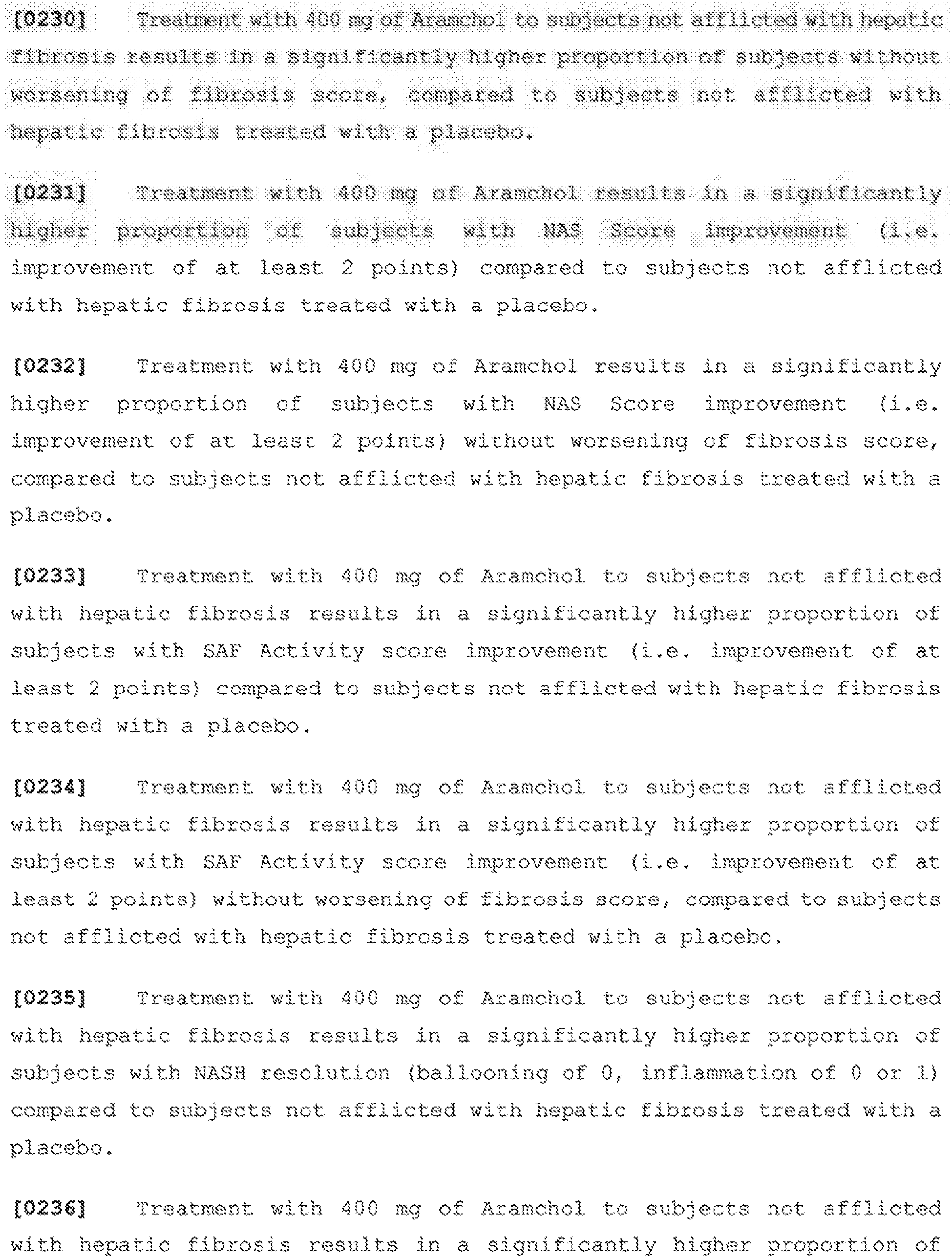

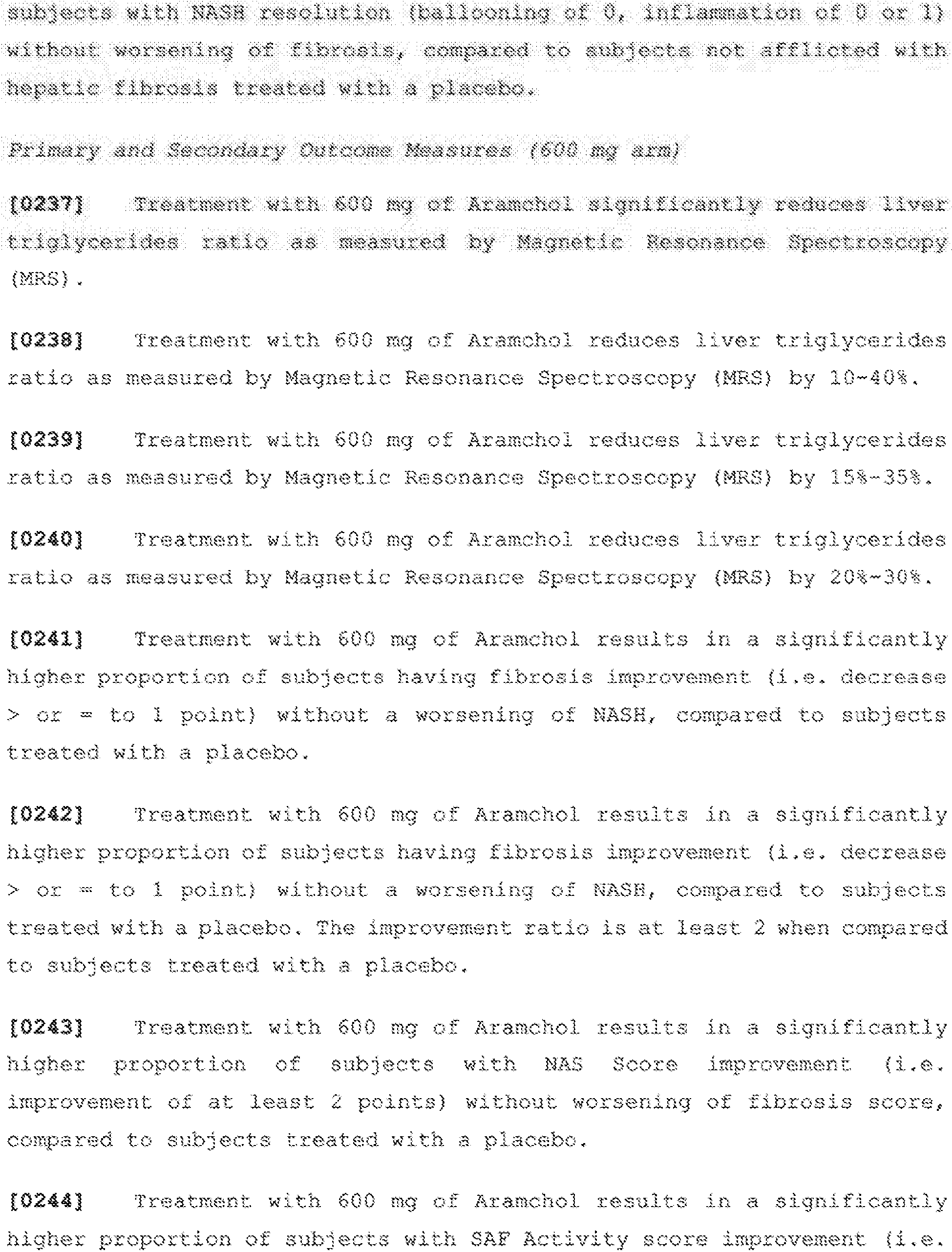

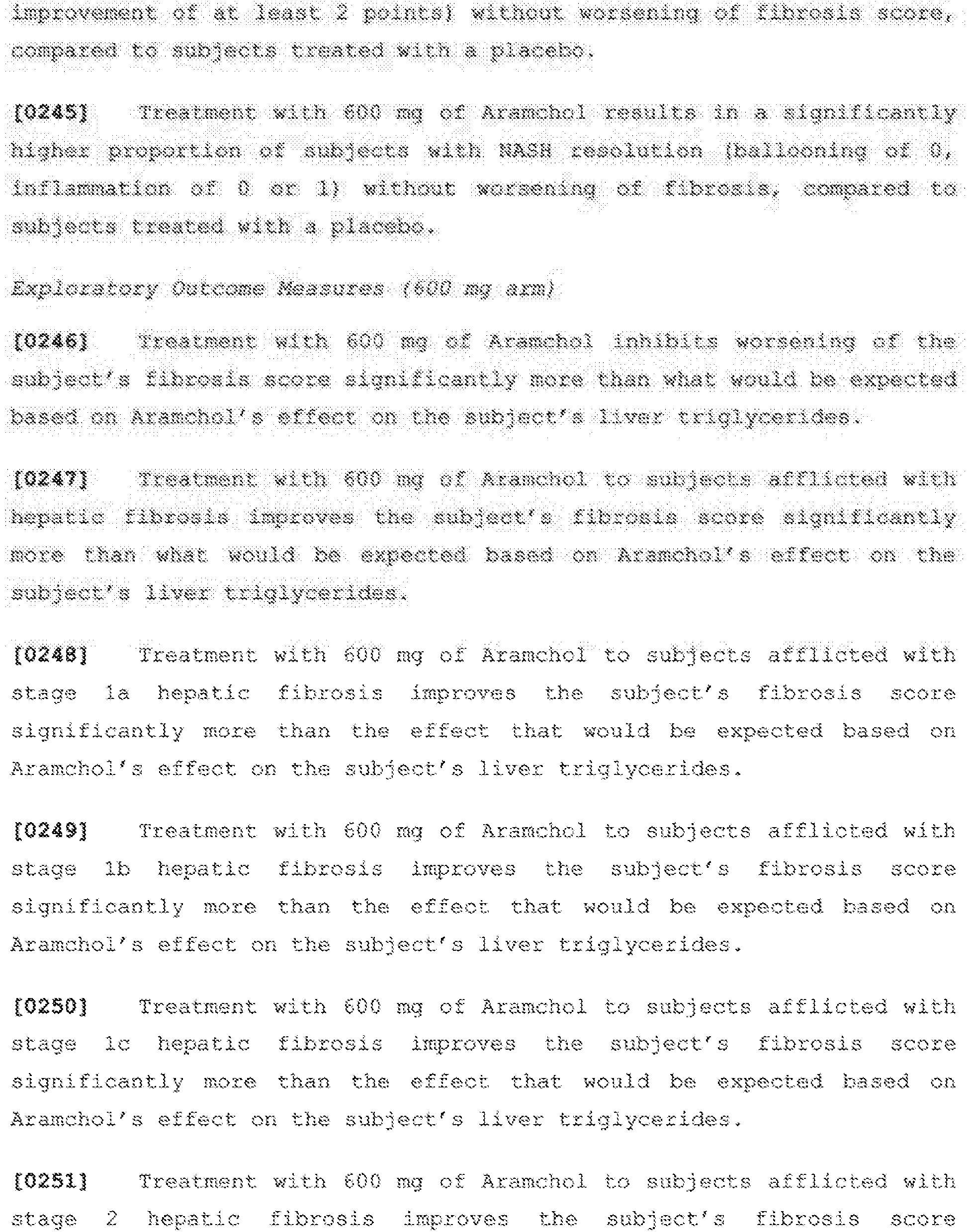

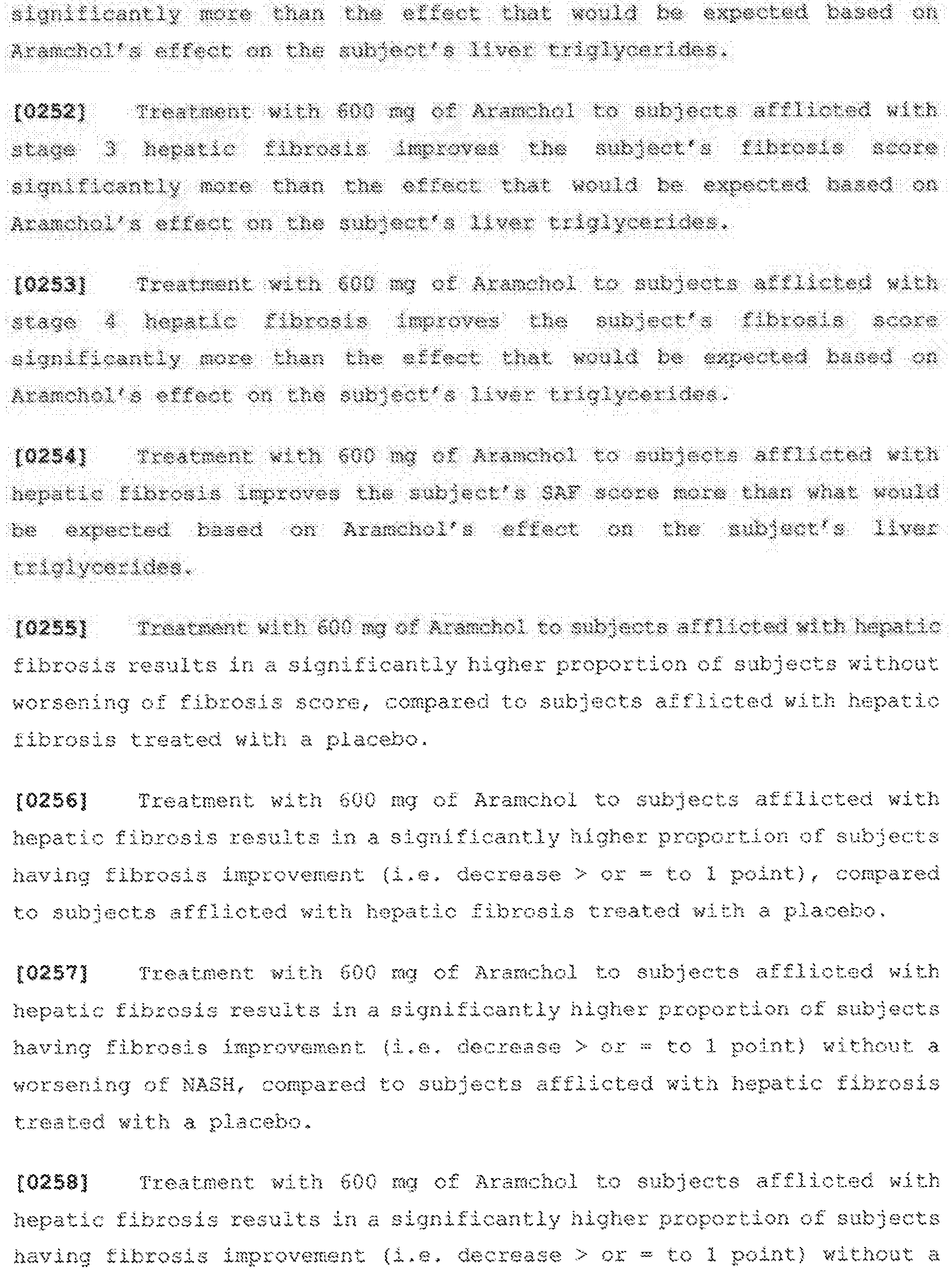

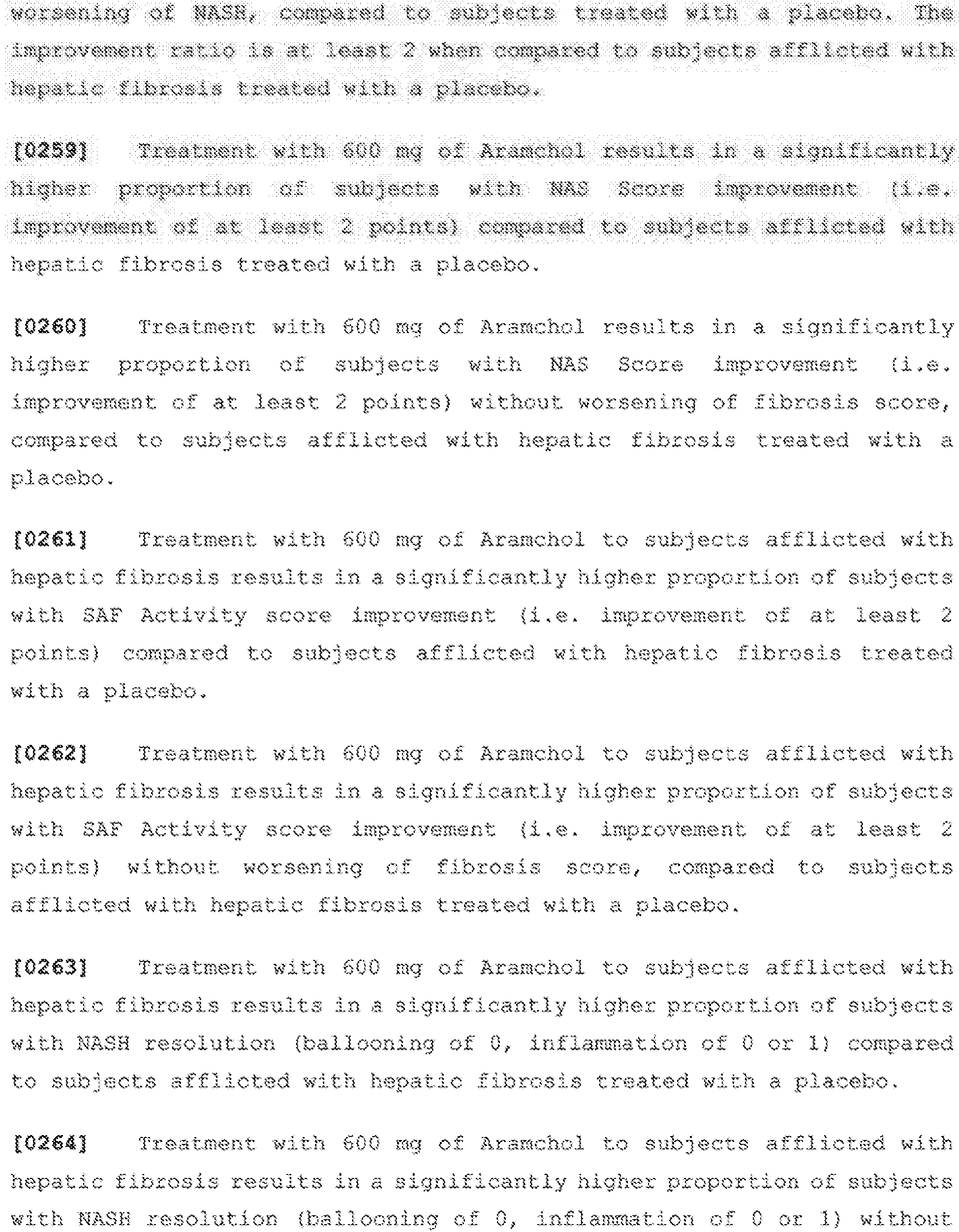

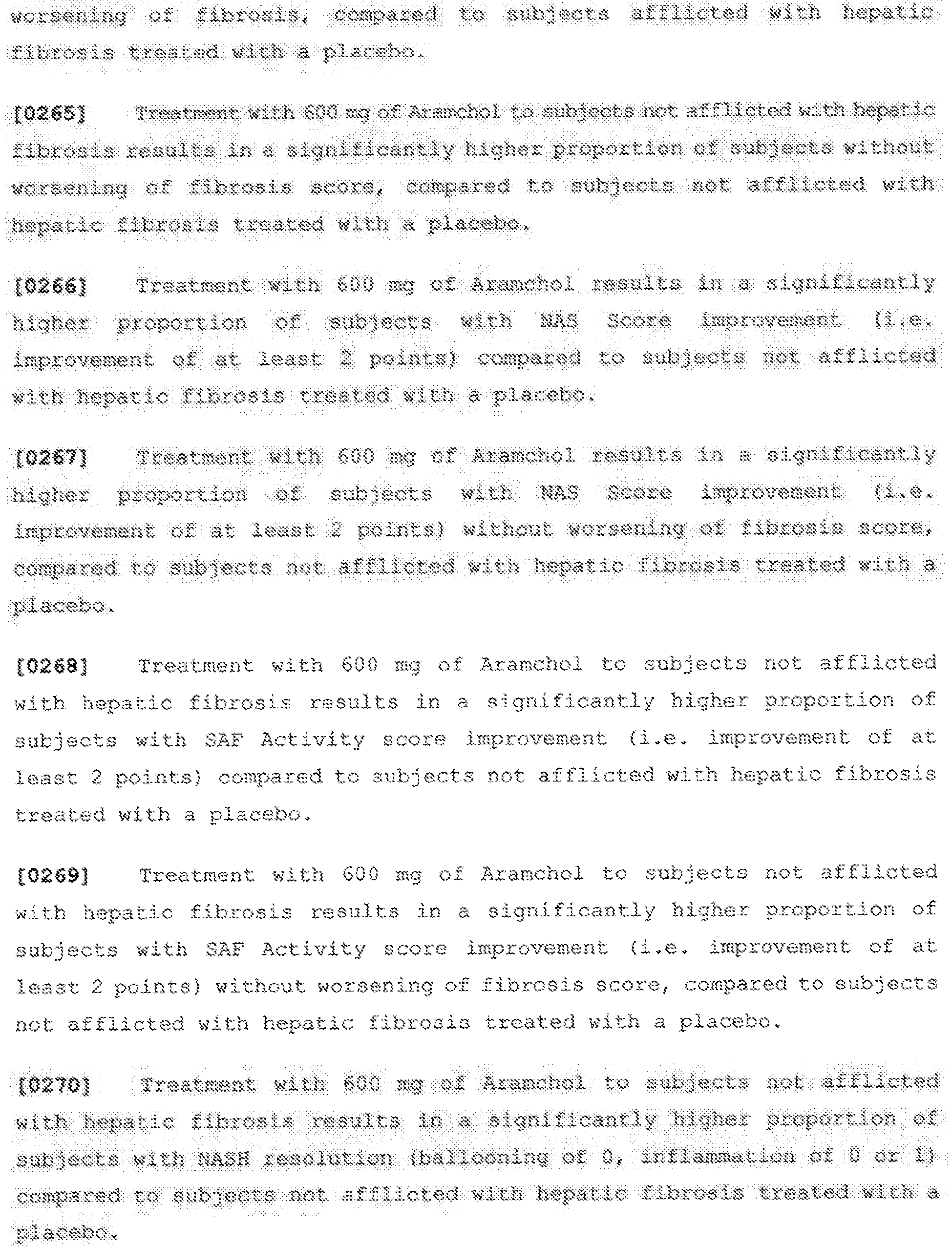

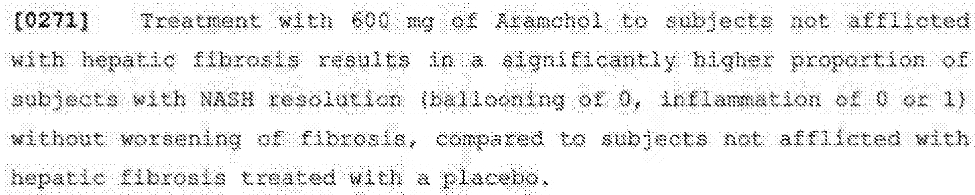

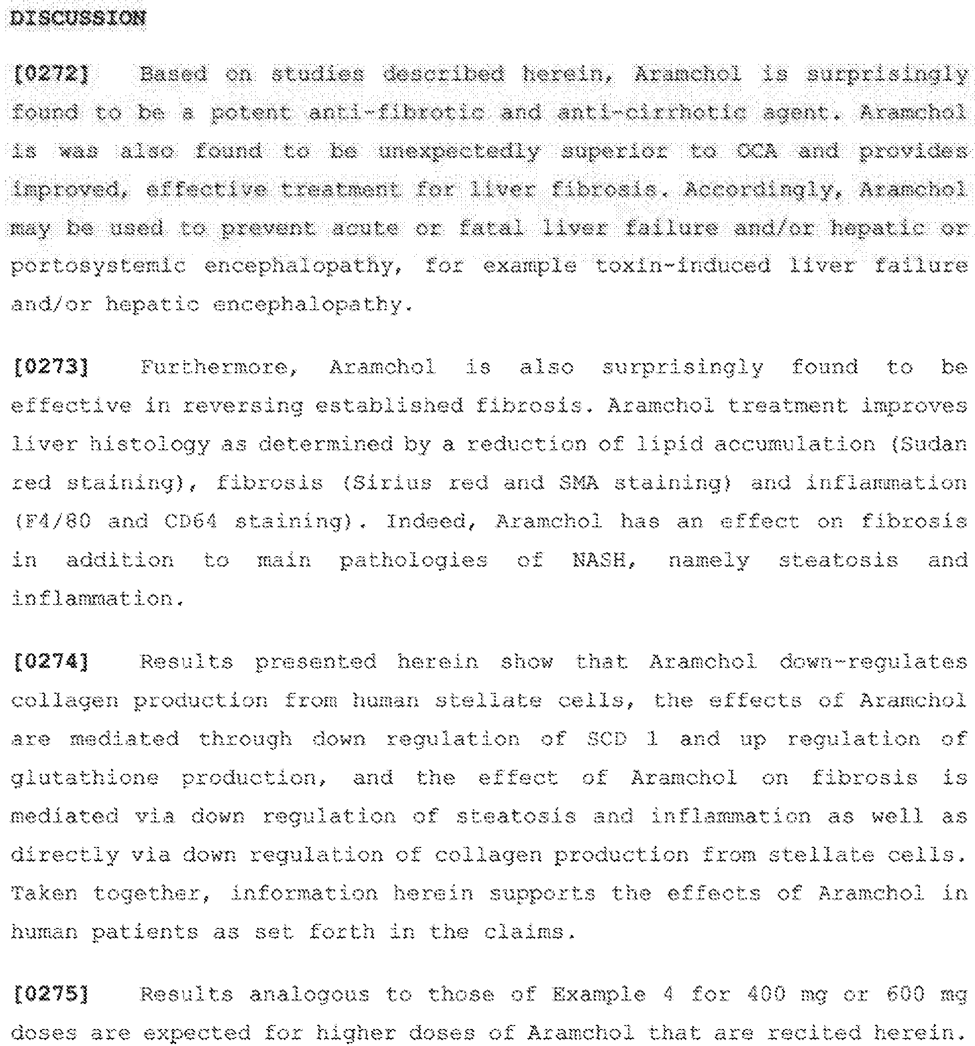

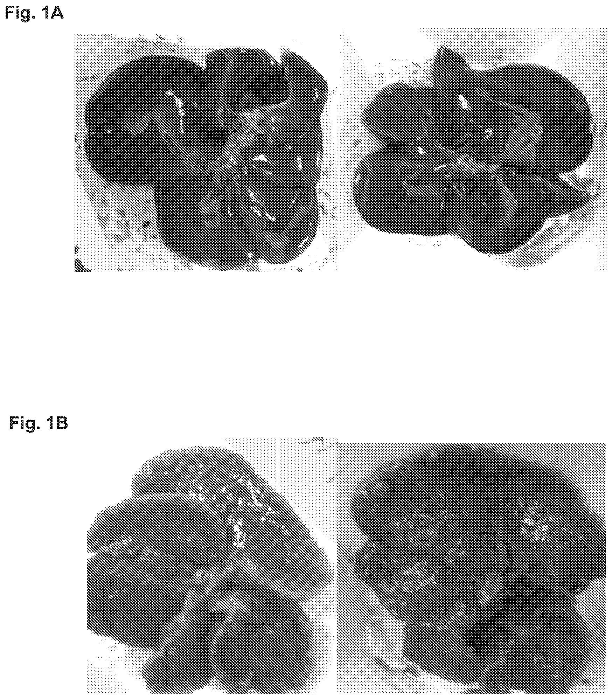

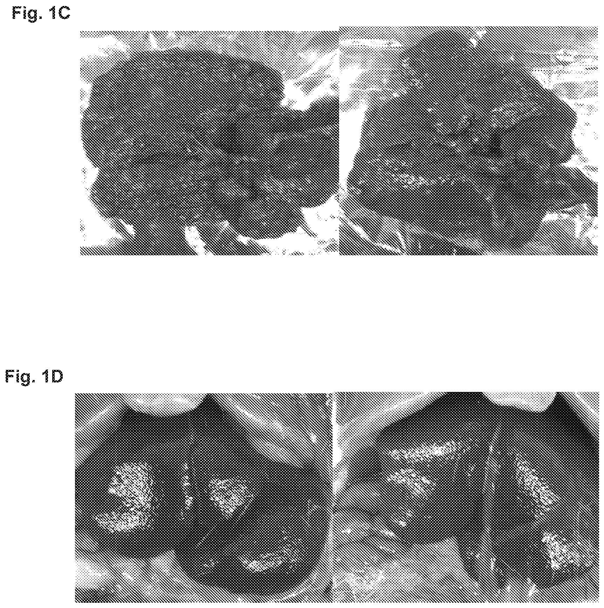

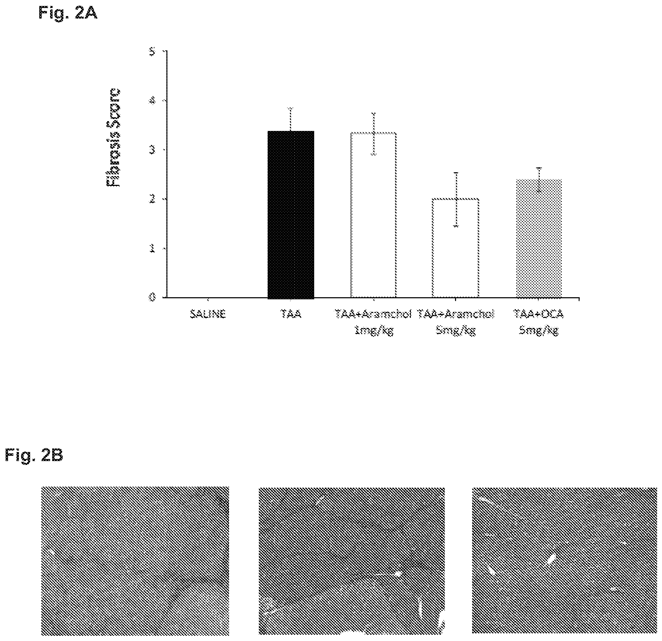

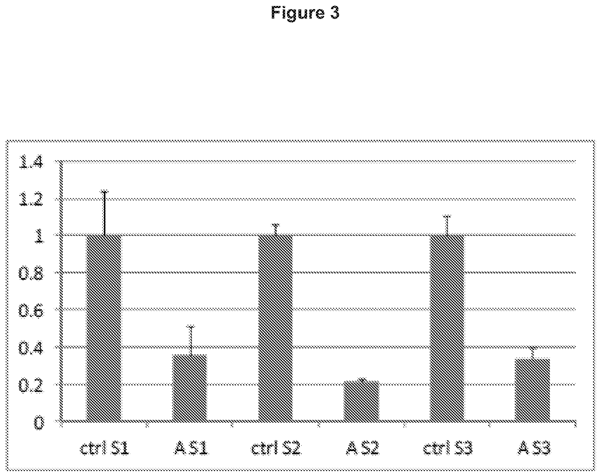

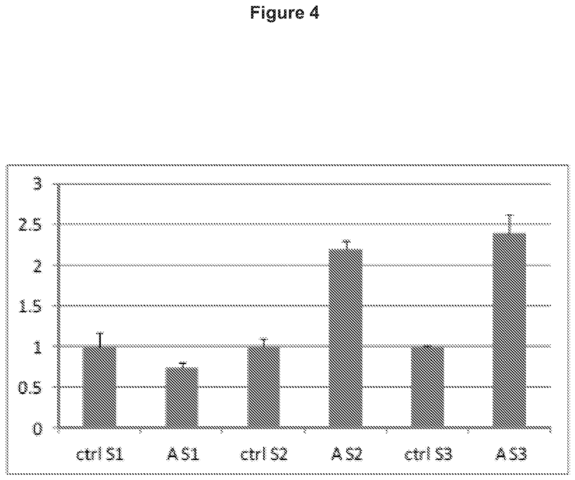

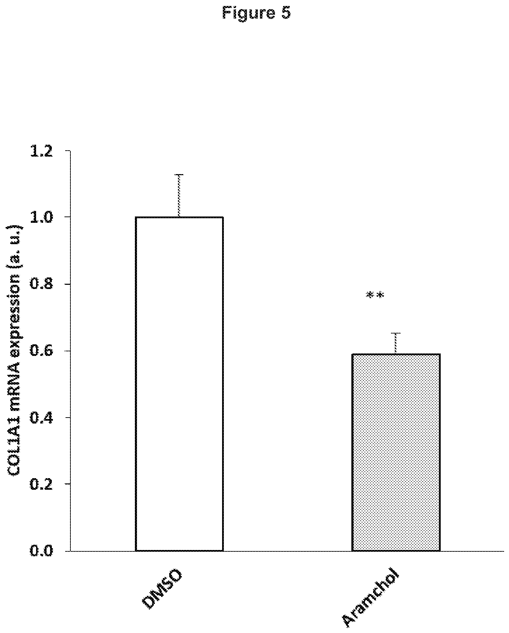

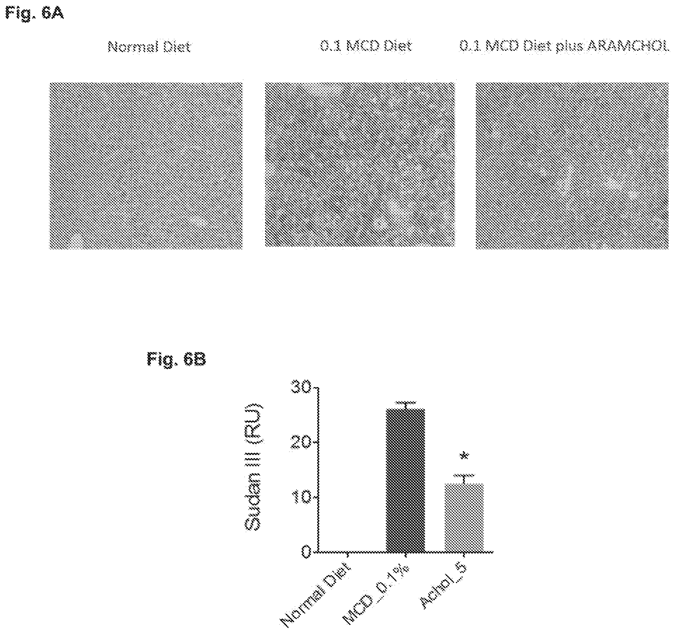

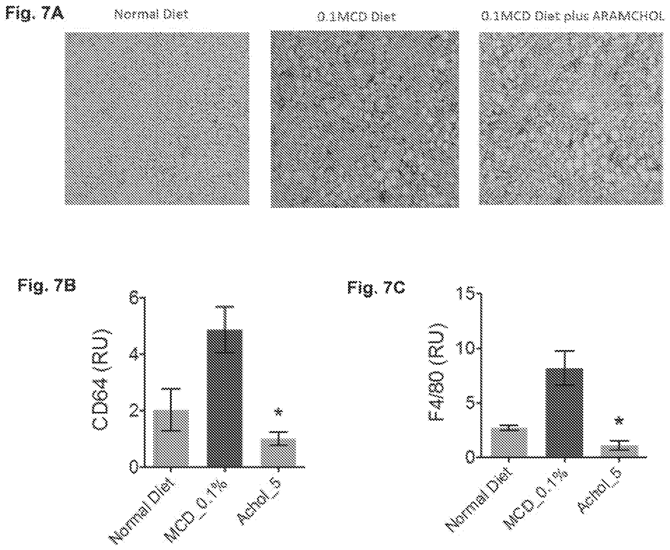

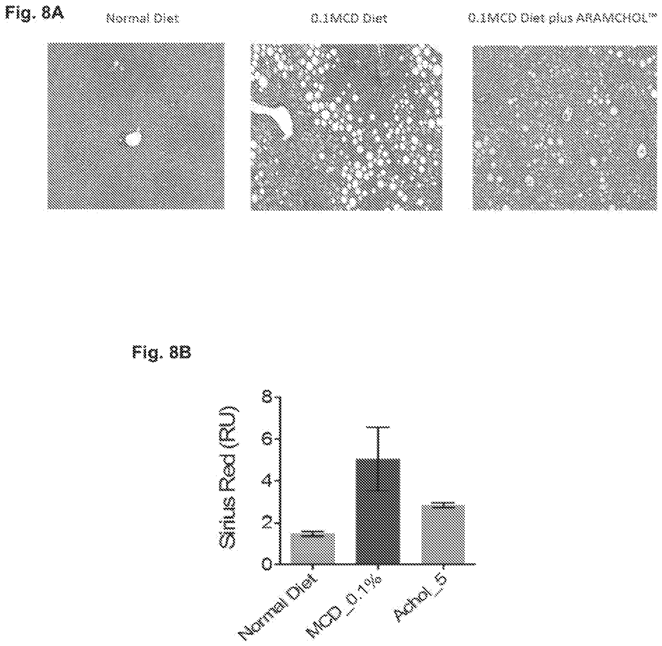

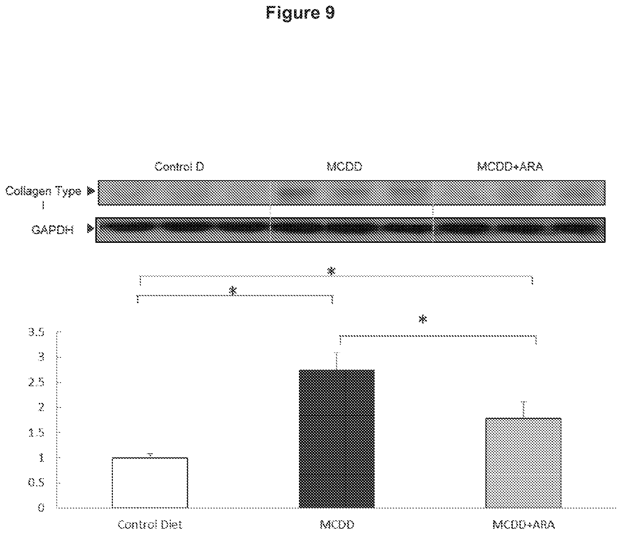

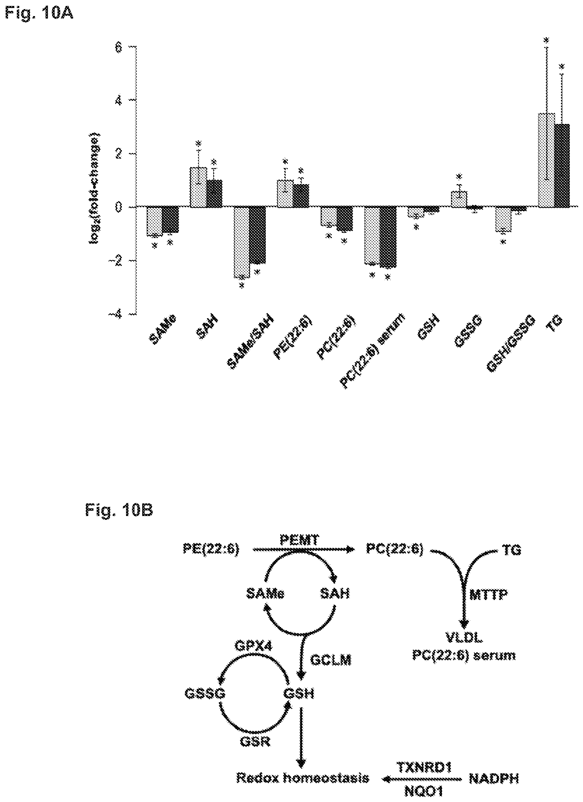

## Claims

1. An oral dose of greater than 300 mg per day of 3β-arachidylamido-7α, 12α-dihydroxy-5β-cholan-24-oic acid (Aramchol), or a pharmaceutically acceptable salt thereof, as the sole active ingredient, for use in inhibiting the development of hepatic fibrosis in a human subject afflicted with Non-Alcoholic Fatty Liver Disease and having a fibrosis score of zero, wherein the Aramchol inhibits collagen synthesis in hepatic stellate cells.

2. The oral dose for use according to claim 1 wherein the human subject is afflicted with Non-Alcoholic Steatohepatitis (NASH).

3. The oral dose for use according to claim 2, wherein the human subject has a NAFLD Activity (NAS) Score of at least 4, at least 5, at least 6, or at least 7; or wherein the human subject has a ballooning score of at least 1, an inflammation score of at least 1, and a steatosis score of at least 1; or wherein the human subject is afflicted with Diabetes Mellitus type II or pre-diabetes, or any combination thereof.

4. The oral dose for use according to any of claims 1-3 wherein between 350 mg and 1200 mg of Aramchol is administered to the subject per day, for example wherein 400 mg or 600 mg of Aramchol is administered to the subject per day.

5. The oral dose for use according to any of claims 1-4 wherein the Aramchol is administered over the course of at least 52 weeks.

6. The oral dose for use according to any of claims 1-5 wherein inhibiting the development of hepatic fibrosis comprises reducing progression of hepatic fibrosis relative to a patient not treated with Aramchol.

7. The oral dose for use according to any of claims 1-6 wherein the human subject is not afflicted with Non-Alcoholic Steatohepatitis (NASH) at commencement of administration and the administration of Aramchol inhibits progression from Non-Alcoholic Fatty Liver Disease (NAFLD) to NASH.

8. The oral dose for use according to any of claims 1-7 wherein the human subject has a diet that is high fat and high calorie and/or is resistant to lifestyle intervention or to diet intervention.

9. The oral dose for use according to any one of claims 1-8, wherein the subject is naïve to Aramchol treatment or wherein the patient is naive to Aramchol treatment and to NAFLD treatment

## Patentansprüche

1. Orale Dosis von täglich mehr als 300 mg 3β-Arachidylamido-7α,12α-dihydroxy-5β-cholan-24-oinsäure (Aramchol) oder von einem pharmazeutisch annehmbaren Salz davon als dem einzigen aktiven Inhaltsstoff zur Anwendung beim Hemmen der Entstehung einer hepatischen Fibrose bei einem menschlichen Subjekt, das an nichtalkoholischer Fettlebererkrankung (NAFLD) leidet und einen Fibrose-Score von Null hat, wobei das Aramchol die Kollagensynthese in hepatischen Stellatzellen hemmt.

2. Orale Dosis nach Anspruch 1, wobei das menschliche Subjekt an nichtalkoholischer Steatohepatitis (NASH) leidet.

3. Orale Dosis nach Anspruch 2, wobei das menschliche Subjekt einen NAFLD-Aktivitäts-Score (NAS) von mindestens 4, mindestens 5, mindestens 6 oder mindestens 7 hat; oder wobei das menschliche Subjekt einen Leberzellschwellungs-Score (Ballooning) von mindestens 1, einen Entzündungs-Score (Inflammation) von mindestens 1 und einen Verfettungs-Score (Steatosis) von mindestens 1 hat; oder wobei das menschliche Subjekt an Diabetes mellitus (Diabetes-Typ-2) oder Vordiabetes oder einer Kombination davon leidet.

4. Orale Dosis nach einem der Ansprüche 1-3, wobei dem Subjekt zwischen 350 mg und 1200 mg Aramchol täglich verabreicht werden, wobei dem Subjekt beispielsweise 400 mg oder 600 mg Aramchol täglich verabreicht werden.

5. Orale Dosis zur Anwendung nach einem der Ansprüche 1-4, wobei das Aramchol im Verlauf von mindestens 52 Wochen verabreicht wird.

6. Orale Dosis zur Anwendung nach einem der Ansprüche 1-5, wobei ein Hemmen der Entstehung einer hepatischen Fibrose ein Reduzieren der Progredienz einer hepatischen Fibrose in Bezug auf einen nicht mit Aramchol behandelten Patienten umfasst.

7. Orale Dosis zur Anwendung nach einem der Ansprüche 1-6, wobei das menschliche Subjekt zu Beginn der Verabreichung nicht an nichtalkoholischer Steatohepatitis (NASH) leidet und wobei die Verabreichung von Aramchol eine Progredienz von nichtalkoholischer Fettlebererkrankung (NAFLD) zu NASH hemmt.

8. Orale Dosis zur Anwendung nach einem der Ansprüche 1-7, wobei das menschliche Subjekt eine fett- und kalorienreiche Ernährungsweise hat und/oder gegen Eingriffe in die normale Lebensführung oder Ernährungsweise resistent ist.

9. Orale Dosis zur Anwendung nach einem der Ansprüche 1-8, wobei das Subjekt in Bezug auf Aramchol behandlungsnaiv ist oder wobei der Patient in Bezug auf NAFLD behandlungsnaiv ist.

## Revendications

1. Dose orale supérieure à 300 mg par jour d'acide 3β-arachidylamido-7α-12α-dihydroxy-5β-cholan-24-oïque (Aramchol), ou d'un sel pharmaceutiquement acceptable de celui-ci, comme seul ingrédient actif, permettant d'inhiber le développement de la fibrose hépatique chez un sujet humain atteint de stéatose hépatique non alcoolique et ayant un score de fibrose de zéro, l'Aramchol inhibant la synthèse de collagène dans les cellules hépatiques étoilées.

2. Dose orale à utiliser selon la revendication 1, dans laquelle le sujet humain est atteint de stéatohépatite non alcoolique (SHNA).

3. Dose orale à utiliser selon la revendication 2, dans laquelle le sujet humain a un score d'activité NAFLD (NAS) d'au moins 4, d'au moins 5, d'au moins 6 ou d'au moins 7 ; ou dans lequel le sujet humain a un score de ballonnement d'au moins 1, un score d'inflammation d'au moins 1 et un score de stéatose d'au moins 1 ; ou dans lequel le sujet humain est atteint de diabète sucré de type II ou de prédiabète, ou de toute combinaison de ceux-ci.

4. Dose orale à utiliser selon l'une quelconque des revendications 1 à 3, dans laquelle entre 350 mg et 1200 mg par jour d'Aramchol sont administrés au sujet, par exemple dans laquelle 400 mg ou 600 mg par jour d'Aramchol sont administrés au sujet.

5. Dose orale à utiliser selon l'une quelconque des revendications 1 à 4, dans laquelle l'Aramchol est administré pendant au moins 52 semaines.

6. Dose orale à utiliser selon l'une quelconque des revendications 1 à 5, dans laquelle l'inhibition du développement de la fibrose hépatique comprend la réduction de la progression de la fibrose hépatique par rapport à un patient non traité avec Aramchol.

7. Dose orale à utiliser selon l'une quelconque des revendications 1 à 6, dans laquelle le sujet humain n'est pas atteint de stéatohépatite non alcoolique (SHNA) au début de l'administration et l'administration d'Aramchol inhibe la progression de la stéatose hépatique non alcoolique (NAFLD) à la SHNA.

8. Dose orale à utiliser selon l'une quelconque des revendications 1 à 7, dans laquelle le sujet humain a un régime riche en graisses et en calories et/ou résiste à une modification de son mode de vie ou à une modification de son régime alimentaire.

9. Dose orale à utiliser selon l'une quelconque des revendications 1 à 8, dans laquelle le sujet n'a jamais reçu de traitement à l'Aramchol ou dans laquelle le patient n'a jamais reçu de traitement à l'Aramchol et de traitement de la NAFLD.
